# EUROPÄISCHE PATENTSCHRIFT

(11) **EP 2 139 396 B1**
(45) Veröffentlichungstag und Bekanntmachung des Hinweises auf die Patenterteilung: **31.08.2016**
(21) Anmeldenummer: 08748934.0
(22) Anmeldetag: 16.04.2008
(51) Int. Cl.: A61B 5/15

(54) **STECHGERÄT UND ANALYSEGERÄT**
PRICKING DEVICE AND ANALYSIS DEVICE
APPAREIL DE PERÇAGE ET APPAREIL D'ANALYSE

(30) Priorität: 18.04.2007 EP 07007891
(43) Veröffentlichungstag der Anmeldung: 06.01.2010
(73) Patentinhaber: Roche Diabetes Care GmbH, 68305 Mannheim (DE); F. Hoffmann-La Roche AG, 4070 Basel (CH)
(72) Erfinder: LIST, Hans, 64754 Hesseneck-Keilbach (DE); HAAR, Hans-Peter, 69168 Wiesloch (DE)
(74) Vertreter: Jany, Peter
(86) Internationale Anmeldenummer: PCT/EP2008/003023
(87) Internationale Veröffentlichungsnummer: WO 2008/128688

(56) Entgegenhaltungen:
- WO-A-2005/104949
- US-A1- 2003 208 140
- US-A1- 2004 162 506

## Beschreibung

Die Erfindung betrifft ein Stechgerät zum Erzeugen einer Einstichwunde in der Haut eines Menschen oder Tieres zum Gewinnen einer Probe einer Körperflüssigkeit mit einem ersten analytischen Verbrauchsmittel, das ein Stechelement umfasst, zum Durchführen einer Analyse eines medizinisch bedeutsamen Bestandteils der Probe mit einem zweiten analytischen Verbrauchsmittel, das ein Testelement umfasst, für Analyse- oder Diagnosezwecke anhand eines medizinisch bedeutsamen Bestandteils der Probe. Das Stechgerät umfasst ein Andruckteil zum Anpressen des Stechgeräts an einen Körperteil, in dem mit dem Stechelement an einer Probenentnahmestelle eine Einstichwunde erzeugt werden soll, aus der eine Probe entnommen werden soll, einen Antrieb zum Antreiben eines in das Stechgerät eingesetzten Stechelements für eine Stechbewegung des Stechelements entlang eines vorbestimmten Einstechweges in eine Stechposition, wobei die Stechbewegung eine schnelle Einstichbewegung umfasst, auf die eine sich unmittelbar anschließende Rückzugsbewegung folgt, ein Gehäuse mit einer Körperteilanlageöffnung für den Körperteil, wobei das Stechelement in einer Ausgangsposition in dem Gehäuse angeordnet ist und an der Körperteilanlageöffnung mittels des Antriebs in die Stechposition in den angelegten Körperteil einstechbar ist, und eine Kontrolleinrichtung zum Überprüfen einer gewonnenen Probe darauf hin, ob die gewonnene Probenmenge ausreicht, um mit einem Testelement die Analyse durchzuführen.

Die entnommene Körperflüssigkeit ist in der Regel Blut. In manchen Fällen handelt es sich aber auch um die Gewinnung einer Probe von interstitieller Flüssigkeit. Nachfolgend wird ohne Beschränkung der Allgemeinheit beispielhaft auf Blut, auch als Beispiel für andere aus einer Einstichwunde gewinnbare Körperflüssigkeiten, Bezug genommen.

Entsprechende Stechsysteme bestehen in der Regel aus zur einmaligen Verwendung vorgesehenen (disposiblen) Stechelementen zum Einstechen in die Haut und dem Stechgerät mit einem Stechelementantrieb für die Stechbewegung des Stechelements. Das Stechgerät eines solchen Stechsystems hat ein Andruckteil zum Anpressen an das Körperteil, in dem eine Einstichwunde erzeugt werden soll, und ein Auslösemittel, durch dessen Betätigung ein Benutzer eine Einstichbewegung eines Stechelements auslösen kann. Alternativ kann die Einstichbewegung auch automatisch durch das Andrücken des Stechgerätes auf die Haut ausgelöst werden. Das Stechelement ist beispielsweise eine Lanzette, eine Nadel oder ein Nadelelement. Derartige Stechelemente können beispielsweise in einem Magazin in ein Stechgerät eingesetzt werden.

Disposible Stechelemente werden seit langem benutzt, um für analytischdiagnostische Zwecke eine geringe Menge Blut aus einem Körperteil (meist aus einem Finger oder Ohrläppchen) zu entnehmen. In diesem Zusammenhang werden die Stechelemente üblicherweise als Lanzetten bezeichnet. Zum manuellen Einstechen vorgesehene Lanzetten werden in der Regel nur von medizinisch trainiertem Personal verwendet. Dennoch ist der Einstich mit erheblichem Schmerz verbunden.

Bereits seit langem werden auch Stechgeräte verwendet, die einen Stechelementantrieb enthalten. Häufig ist eine regelmäßige Überwachung bestimmter analytischer Werte des Blutes erforderlich. Stechgeräte werden insbesondere von Diabetikern verwendet, die mehrmals täglich mittels einer Blutzucker-Selbstkontrolle ihren Blutzuckerspiegel überprüfen müssen, um durch Anpassung von Insulininjektionen an den stark schwankenden Bedarf ihren Blutzuckerspiegel möglichst ständig innerhalb bestimmter Sollgrenzen zu halten. Entsprechend ist auch die Überprüfung von Blutgerinnungsparametern durch eine Patienten-Blutgerinnungs-Selbstkontrolle verbreitet. Das Stechgerät kann disposibel, d.h. zum nur einmaligen Gebrauch für das Gewinnen einer Probe bestimmt, mit einer fest integrierten Lanzette ausgebildet sein. In der Regel ist es jedoch mehrfach verwendbar und hat eine Halterung, mittels der jeweils eine Lanzette auswechselbar mit dem Stechantrieb gekoppelt werden kann. Da die Geräte und Lanzetten aneinander angepasste, von dem gleichen Hersteller gelieferte Elemente sind, werden sie als "Stechsystem" oder "Blutentnahmesystem" bezeichnet.

Als Antriebselement für den in einem Gehäuse des Stechgerätes angeordneten Lanzettenantrieb dient meist eine Feder oder ein elektromagnetischer Antrieb. Durch eine Lanzettenführung wird gewährleistet, dass die Stechbewegung auf einem vorbestimmten Einstichweg erfolgt. Dabei wird die Bewegung der Lanzette in Richtung auf die Hautoberfläche bis zu dem Umkehrpunkt (Vortriebsphase der Stechbewegung) angetrieben, beispielsweise mittels eines Federsystems oder eines elektromagnetischen Antriebs, und ein entsprechender Antrieb kann für die Rückzugsbewegung der Lanzette (Rückzugsphase der Stechbewegung) vorgesehen sein.

Bei den üblichen Konstruktionen haben die Stechgeräte an ihrem in Einstichrichtung des Stechelements vorderen Ende eine Austrittsöffnung, aus der die Spitze der Lanzette nur für kurze Zeit austritt, um eine Wunde in einem Körperteil zu erzeugen, gegen das das vordere Ende des Stechgerätes gedrückt wird. Die Stechtiefe ist dabei durch den Abstand in Einstichrichtung zwischen der Position, die die Lanzettenspitze am Umkehrpunkt der Lanzettenbewegung erreicht und der Ebene einer Hautkontaktfläche definiert, die die Austrittsöffnung ringförmig umgibt und im Moment des Einstichs an der Haut anliegt. Das vordere Ende des Stechgerätes mit der Hautkontaktfläche bildet also ein Stechtiefenreferenzelement, durch das gewährleistet wird, dass die Stechtiefe einem vorgegebenen Wert entspricht.

Um die Stechtiefe zu kontrollieren, ist es gebräuchlich, den Bewegungsweg der Lanzette in Einstichrichtung zu begrenzen, beispielsweise durch ein mit der Lanzette verbundenes Anschlagteil, das auf eine korrespondierende Anschlagfläche im Gehäuse des Stechgerätes auftrifft.

Derartige Blutentnahmesysteme müssen hohe Anforderungen erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Aktivität etc. stark schwankt) ihren Blutzuckerspiegel möglichst ständig innerhalb bestimmter Sollgrenzen zu halten. Durch umfangreiche wissenschaftliche Untersuchungen wurde bewiesen, dass mittels einer Intensivtherapie mit mindestens vier Blutanalysen pro Tag ein dramatischer Rückgang schwerster Spätschäden des Diabetes mellitus (beispielsweise einer Retinopathie mit resultierender Erblindung des Patienten) erreicht werden kann. Diese Intensivtherapie setzt voraus, dass die häufige Blutentnahme mit einem möglichst geringen Schmerz verbunden ist.

Ein ständiges Ziel in der Entwicklung von Stechsystemen besteht folglich darin, mit möglichst geringem Schmerz eine Einstichwunde zu erzeugen, aus der sich eine brauchbare Probe, d.h. eine ausreichende Menge einer Körperflüssigkeit, gewinnen lässt. Sowohl für das Schmerzempfinden als auch für die Probengewinnung ist die Einstichtiefe von großer Bedeutung. Generell gilt, dass das Schmerzempfinden mit zunehmender Einstichtiefe ebenso wie die Flüssigkeitsmenge, die sich aus der Einstichwunde gewinnen lässt, zunimmt. An Stechgeräte besteht deshalb die Anforderung, nur eine möglichst geringe Menge einer Blutprobe zu entnehmen, die für die Durchführung der Analyse ausreicht, und die Einstichtiefe einerseits so gering wie möglich, andererseits so tief wie nötig zu gestalten, und es gibt verschiedene Entwicklungen, um die Blutentnahme möglichst schmerzfrei zu gestalten.

In der Praxis wird von einem Blutentnahmesystem jedoch nicht nur erwartet, dass es der Anforderung an ein minimales Schmerzempfinden genügt, sondern gleichzeitig soll es eine einfache Bedienung aufweisen, eine kompakte schlanke Bauweise haben und eine einfache, kostengünstige Konstruktion ermöglichen. Aus diesen praktischen Anforderungen heraus wurden und werden Blutanalysegeräte entwickelt, die diesen teilweise gegenläufigen Anforderungen möglichst weitgehend genügen.

Da die Einstichtiefe auf einen möglichst geringen Wert eingestellt wird, kann es in der Praxis vorkommen, dass bei einem Einstich keine oder keine ausreichende Blutprobe gewonnen wird. Es werden daher im Stand der Technik Anstrengungen unternommen, um das Risiko eines erfolglosen Einstichs zu reduzieren. Beispielsweise sind Stechgeräte mit einem Drucksensor bekannt, bei denen automatisch eine Einstichbewegung ausgelöst wird, sobald auf dem Andruckteil ein Druck lastet, der einen vorgegebenen Mindestdruck übersteigt.

Während in Kliniken und bei niedergelassenen Ärzten oftmals durch eine Venenpunktion mehrere Milliliter Blut einer zu untersuchenden Person für die Analyse gewonnen werden, um damit eine Vielzahl von Labortests durchführen zu lassen, reichen für einzelne Analysen, die gezielt auf einen Parameter gerichtet sind, heutzutage oft wenige µl Blut aus. Die Gewinnung von geringen Probenmengen im Bereich weniger µl oder weniger zum Bestimmen analytischer Parameter ist insbesondere zur Messung des Blutglukosespiegels verbreitet, findet aber auch beispielsweise Anwendung bei der Bestimmung von Gerinnungsparametern, Triglyceriden, HBA 1c oder Lactat.

Solch geringe Blutmengen erfordern keine Venenpunktion sondern können mit Hilfe einer sterilen, scharfen Lanzette, die durch die Haut, z.B. in die Fingerbeere oder das Ohrläppchen der zu untersuchenden Person gestoßen wird, gewonnen werden. Insbesondere eignet sich diese Methode, wenn die Analyse der Blutprobe unmittelbar nach der Blutgewinnung durchgeführt werden kann.

Die zum Entnehmen von Körperflüssigkeit aus einem Körperteil durch Erzeugen einer kleinen Stichwunde verwendeten Lanzetten haben zumeist eine metallene Lanzettennadel, deren Spitze angeschliffen sein kann.

Diese Lanzetten müssen bis zu ihrer Verwendung steril aufbewahrt werden und sollen nach ihrer Verwendung vorzugsweise so entsorgt werden, dass sie nicht zu Verletzungen führen können. Es wurden daher Blutentnahmesysteme vorgeschlagen, bei denen die Lanzetten in einem Lanzetten-Vorratsbehältnis, in dem eine Mehrzahl von Lanzetten zum Entnehmen aus dem Lanzetten-Vorratsbehältnis an einer Entnahmeposition vorrätig gehalten wird.

Eine mögliche Ausführungsform eines derartigen Lanzetten-Vorratsbehältnis ist ein Trommelmagazin, aus dem die Lanzetten einzeln entnehmbar sind, wobei die Lanzetten in Kammern in dem Trommelmagazin angeordnet sind, die jeweils einzeln verschlossen sind. Die gebrauchten Lanzetten werden dabei entweder außerhalb des Gerätes bzw. des Analysegerätes entsorgt oder können nach ihrem Gebrauch auch zum sicheren Entsorgen in das Lanzetten-Vorratsbehältnis zurückgeführt werden.

Die Erfindung betrifft auch ein Analysegerät zum Analysieren eines medizinisch bedeutsamen Bestandteils einer Körperflüssigkeit, beispielsweise Blut oder interstitielle Flüssigkeit. Dabei wird eine qualitative oder quantitative Analyse durchgeführt, also beispielsweise das Vorhandensein, das Nichtvorhandensein oder die Konzentration eines bestimmten Analyten in einer Probe bestimmt, wobei die Probe mit einem oben beschriebenen Stechgerät gewonnen wird, das in das Analysegerät integriert ist. Das Analysegerät umfasst also ein erfindungsgemäßes Stechgerät, wobei in das Analysegerät eine Analyseeinrichtung zum Durchführen einer Analyse oder Diagnose mittels der von dem Stechgerät gewonnenen Probe integriert ist. Die Erfindung bezieht sich insbesondere auf ein tragbares, netzunabhängig betreibbares und von einem Patienten bedienbares Analysegerät für die Patienten-Selbstkontrolle, den so genannten Bereich des "Home-Monitoring", insbesondere ein Blutglukosemessgerät, ein Cholesterin-Messgerät oder ein Blutgerinnungsparameter-Messgerät.

Ferner betrifft die Erfindung ein entsprechendes Verfahren zum Vorbereiten eines Stechgerätes für das manuelle Aufnehmen einer Probe.

Ein Blutglukosemessgerät ist ein Messgerät, mit dessen Hilfe der Blutzuckergehalt qualitativ oder quantitativ bestimmt werden kann. In der Regel wird hierzu in einem Körper eine Einstichwunde erzeugt, ein Bluttropfen entnommen, der Bluttropfen auf das Testelement aufgebracht und mit Hilfe des Testelements und des Blutglukosemessgeräts der Blutglukosegehalt in dem Tropfen bestimmt.

Die Erfindung bezieht sich also nicht nur auf Stechsysteme bzw. Stechgeräte, die ausschließlich dazu dienen, einen Bluttropfen für eine anschließende Analyse mit einem anderen Gerät zu gewinnen. Vielmehr richtet sie sich insbesondere auch auf so genannte integrierte Systeme, mit denen nicht nur die Blutentnahme, sondern auch die Analyse durchgeführt wird, möglichst ohne zusätzliche Handhabungsschritte durch den Benutzer. Damit sind zusätzliche Anforderungen verbunden, die unter anderem aus der räumlichen Enge resultieren, wenn beide Funktionen in einem (aus Handhabungsgründen möglichst kleinen) Gerätegehäuse untergebracht werden müssen.

Derartige Analysegeräte umfassen ein Gerätegehäuse, eine in dem Gerätegehäuse angeordnete Messeinrichtung zum Durchführen der Analyse an einer mit dem Stechgerät gewonnenen Probe und einen Prozessor mit Software zum Verarbeiten der von der Messeinrichtung ermittelten Messwerte und zum Aufbereiten der Analysemessdaten aus den Messwerten, in der Regel unter Berücksichtigung von Kalibrationswerten.

Zur qualitativen und quantitativen Analyse von Bestandteilen einer flüssigen Probe, insbesondere einer Körperflüssigkeit von Menschen oder Tieren, werden in großem Umfang Testverfahren eingesetzt, die mit Testelementen arbeiten. Die Testelemente enthalten Reagenzien. Zur Durchführung einer Reaktion wird das Testelement mit der Probe in Kontakt gebracht. Die Reaktion von Probe und Reagenz führt zu einer für die Analyse charakteristischen Veränderung des Testelements, die mit Hilfe eines geeigneten Analysegerätes ausgewertet wird. Das Analysegerät ist in der Regel zum Auswerten eines ganz bestimmten Typs von Testelementen eines bestimmten Herstellers geeignet. Die Testelemente und das Analysegerät bilden wechselseitig aufeinander abgestimmte Bestandteile und werden insgesamt als Analysesystem bezeichnet.

Es sind zahlreiche unterschiedliche Testelement-Typen bekannt, die sich durch das Messprinzip und die verwendeten Reagenzien sowie durch ihren Aufbau unterscheiden. Die Verwendung von Magazinen für Testelemente und/oder Stechelemente ist in diesem Zusammenhang bekannt.

Hinsichtlich des Messprinzips sind kolorimetrische Analysesysteme besonders weit verbreitet. Bei ihnen führt die Reaktion der Probe mit den in dem Testelement enthaltenen Reagenzien zu einer Farbänderung, die visuell oder mittels einer photometrischen Messreinrichtung gemessen werden kann. Daneben haben elektrochemische Analysesysteme eine große Bedeutung erlangt, bei denen die Reaktion der Probe mit den Reagenzien des Testelements zu einer elektrisch messbaren Änderung (einer elektrischen Spannung oder eines elektrischen Stromes) führt, die mit einer entsprechenden Messelektronik gemessen wird. Derartige Analysesysteme werden auch als amperometrische Systeme bezeichnet.

Hinsichtlich des Aufbaus der Testelemente sind insbesondere streifenförmige Testelemente (so genannte Teststreifen) gebräuchlich, die im wesentlichen aus einer länglichen Tragschicht aus Kunststoffmaterial und darauf angebrachten Testfeldern bestehen. Die Testfelder bestehen in der Regel aus einer oder mehreren Reagenzien enthaltenden Testschichten. Solche Teststreifen werden in großem Umfang insbesondere für Blut- und Urinuntersuchungen verwendet.

Bei einem zweiten Typ von Testelementen ist ein Testfeld ähnlich wie ein photographisches Diapositiv von einem Rahmen umgeben. Das Testfeld dieses Testelementtyps besteht in der Regel aus einer oder mehreren Testschichten, die von dem Rahmen gehalten werden und für kolorimetrische Tests geeignete Reagenzien enthalten. Nach Aufgabe der Probe auf das Testfeld und Ablauf der Testreaktion kann die Farbbildung beobachtet bzw. photometrisch vermessen werden.

Vor allem im Bereich des so genannten "Home-Monitoring", also dort, wo medizinische Laien selbst einfache Analysen des Bluts durchführen, und dort insbesondere für die regelmäßige, mehrmals täglich durchzuführende Blutgewinnung durch Diabetiker für die Kontrolle der Blutglukosekonzentration, werden Lanzetten und dazu passende Geräte, so genannte "Stechhilfen", angeboten, die eine möglichst schmerzarme und reproduzierbare Blutgewinnung ermöglichen, und es ist hier wichtig, dass eine einfache und zuverlässige Bedienung des Blutglukosemessgeräts möglich sowie eine informative und verlässliche Bestimmung und Darstellung der Messergebnisse vorhanden ist.

Die gebräuchlichen Analysegeräte sind so genannte Stand-Alone-Messgeräte. Diese Geräte arbeiten autonom, selbständig und unabhängig. Sie haben also ein Display, eine Messeinrichtung, eine Stromversorgung und ein vollständiges User-Interface, das z.B. eine Tastatur, eine Anzeige, einen Signalgeber oder eine Benutzerführung umfassen kann. Der Anwendungszweck und die Eigenschaften derartiger Geräte sind, bis auf gelegentliche Anpassungen der Firmware, festgelegt.

So geht beispielsweise ein bekanntes Gerätekonzept für Blutanalysegeräte davon aus, dass "integrierte Disposables" verwendet werden, bei denen es sich um eine integrierte Kombination von jeweils einem analytischen Verbrauchsmittel (Disposable) in Form eines Stechelements (Nadelelement zum Durchführen des Einstichs) und einem analytischen Verbrauchsmittel (Disposable) in Form eines Testelements (z.B. Testchemie zum Durchführen der Analyse oder Diagnose) handelt. Dabei ist also jeweils für ein Testelement ein Stechelement vorgesehen bzw. vorhanden, das in das Testelement integriert ist. Bei einem integrierten Disposable sind also das erste und das zweite analytische Verbrauchsmittel in einem analytischen Verbrauchsmittel integriert, das sowohl ein Stechelement zum Durchführen des Einstichvorgangs für das Gewinnen der Probe einer Körperflüssigkeit als auch ein Testelement zum Durchführen der Analyse eines medizinisch bedeutsamen Bestandteils der Probe umfasst. Je nach Schwerpunkt der Sichtweise spricht man hierbei auch von einem Stechelement mit integriertem Testelement oder von einem Testelement mit integriertem Stechelement.

Die Übertragung des mit dem Stechelement gewonnen Bluttropfens zu einem Analysesensor bzw. Testelement erfolgt beispielsweise mit Hilfe eines in dem Gerätegehäuse verlaufenden Kapillarkanals. Dies ist ein Beispiel für ein Stechsystem, dessen Nadelelement einen Kapillarkanal aufweist, durch den eine Körperflüssigkeit aus der Haut in das Innere der Stecheinheit transportiert werden kann.

Ein weiteres Beispiel eines solchen Stechsystems ist in der US-Patentanmeldung US 2003/0018282 A1 beschrieben. Dabei umfasst die Stecheinheit nicht nur die in die Haut einzustechende Nadel mit einem zum Transport der Probe geeigneten Kapillarkanal, sondern auch einen Reagenzien enthaltenden Nachweisbereich. Eine solche Stecheinheit, die gleichzeitig einen (beispielsweise als kapillaraktive Saugschicht und/oder Hohlkammer ausgebildeten) Aufnahmebereich für die Probe hat und vorzugsweise auch die für die Analyse erforderlichen Reagenzien enthält, wird auch als "Mikrosampler" bezeichnet. Hinsichtlich näherer Einzelheiten über Mikrosampler wird auf die erwähnte US-Patentanmeldung und die darin zitierten Dokumente, insbesondere das Patent US 5,801,057 verwiesen. Abgesehen von den hier beschriebenen Besonderheiten können im Rahmen der vorliegenden Erfindung Mikrosampler unterschiedlicher Konstruktionen eingesetzt werden.

Die vorliegende Erfindung richtet sich generell auf Stechsysteme unterschiedlicher Typen, wobei das Nadelelement, das in die Haut eingestochen wird, als massive Nadel (wie bei den erwähnten Lanzetten) oder als Kapillarnadel (mit offener Kapillare oder als geschlossene Hohlnadel) ausgebildet sein kann. Soweit auf Lanzetten oder andere speziellere Ausführungsformen Bezug genommen wird, geschieht dies beispielhaft und ohne Beschränkung der Allgemeinheit. Die erläuterten Sachverhalte gelten grundsätzlich auch für andere Ausgestaltungen.

Die Erfindung eignet sich insbesondere für integrierte Systeme, bei denen die Funktionen der Blutgewinnung und der Analyse in einem Gerät vereinigt sind. Im Falle eines Mikrosamplers erfolgt diese Integration vorzugsweise dadurch, dass in dessen Probenaufnahmebereich die für die Analyse erforderlichen Reagenzien und sonstigen Komponenten vorhanden sind. Insoweit unterscheiden sich für die Erfindung geeignete Mikrosampler nicht von bekannten Systemen. Auch die Stechelemente ohne Kapillarkanal können in Verbindung mit der Erfindung in vorteilhafter Weise in integrierten Systemen verwendet werden, wobei in diesem Fall das Stechelement nach dem Einstichvorgang rasch zurückgezogen wird, so dass die aus der Haut austretende Probenflüssigkeit in einen Kapillarkanal eines Analyseelementes eindringen kann, das zu diesem Zweck innerhalb des Stechgerätes in Kontakt mit der aus der Haut austretenden Probenflüssigkeit gebracht wird.

Es können auch integrierte Disposables verwendet werden. Integrierte Disposables sind Verbrauchselemente, die sich dadurch auszeichnen, das sie sowohl ein Stechelement zum Durchführen des Einstichs für das Gewinnen einer Körperflüssigkeitsprobe, beispielsweise einer Blutprobe, als auch ein Testelement, z.B. eine Testchemie zum Durchführen der Analyse eines medizinisch bedeutsamen Bestandteils der Probe beinhalten. Solche Disposables werden häufig in geeigneten Magazinen, die z.B. die Form eines Streifens haben, verwendet.

Die Erfindung bezieht sich also in Bezug auf Analysegeräte allgemein auf jede Kombination analytischer Verbrauchsmittel in Form von Stechelementen, Testelementen und integrierten Disposables. Der Bluttransport bzw. der Transport mittels des Einstichs mit dem Stechelement gewonnener Körperflüssigkeit von der Einstichstelle zu dem Testelement kann auf jede bekannte Weise erfolgen, beispielsweise durch eine Aufnahme mit dem Stechelement selbst mit anschließendem Transport von dem Stechelement zu dem Testelement, z.B. in einer Kapillare, oder durch eine Aufnahme mit dem Testelement direkt, wobei nach dem Stechen mit dem Stechelement mit einem geeigneten Antrieb das Testelement mit der Einstichstelle in Kontakt gebracht wird.

In der Patentliteratur gibt es eine Reihe von integrierten Tests zur Blutanalyse. Sie beschreiben aber meist nur den Testaufbau (US 6,607,658 und EP 1402812 A1 als Beispiele für Testelemente mit integrierter Lanzette, oder US 2002/0168290 A1 als Beispiel für Sampler mit integrierter Testchemie bzw. integriertem Sensor). Einige Veröffentlichungen befassen sich mit Antrieben für das Stechelement, was von einfachen ballistischen Federmechaniken (US 2006/0178600 A1) bis zum elektrischen Direktantrieb reicht. Darüber hinaus befassen sich einige Veröffentlichungen mit Methoden, das Austreten von Blut durch bestimmte Bewegungsprofile zu unterstützen (z.B. US 7,025,774).

Integrierte Disposables sind beispielsweise auch in den Dokumenten US 6,607,658 oder US 2002/0168290 A1 beschrieben. Nach einem Einstichvorgang durch ein Nadelelement erfolgt anschließend automatisch eine Probenaufnahme, die entweder direkt durch das Testelement erfolgen kann, siehe z.B. US 6,607,658 oder durch eine Kapillarstruktur der Nadel, wobei das Blut anschließend zum Testfeld hingeleitet wird. Ein integriertes System mit entsprechenden Antriebseinheiten beschreibt z.B. die US 7,025,774.

Im Alltag ist die Situation nicht selten, dass nach einem Einstich in die Haut keine oder keine ausreichende Probe gewonnen werden kann, sei es dass die Handhabung fehlerhaft war, sei es, dass die korrekte Tiefeneinstellung für den Stich noch nicht ermittelt wurde.

Bevor das Blut jedoch durch das Disposable aufgenommen werden kann, muss zuvor eine automatische Blutexpression erfolgen, die meist durch einen so genannten Fingerkonus oder Ring erfolgt, der entsprechend Druck auf die Fingerkuppe ausübt, so dass der Blutexpressionsschritt unterstützt wird. Eine Blutaufnahme kann so bereits während des Stechvorgangs z.B. durch das Nadelelement unterhalb der Haut erfolgen, oder anschließend von der Hautoberfläche. In der Praxis zeigt sich, dass der Erfolg des Blutexpressionsschrittes sowie der Blutaufnahme häufig von vielen weiteren Faktoren (Elastizität der Haut, Hauttemperatur etc.) abhängt, so dass die zur Zeit zu erreichende "Erfolgsrate" des Expressions- bzw. Blutsammelvorgangs nicht bei 100% liegt. Da üblicherweise die Disposables nur zur Einmalverwendung vorgesehen sind, wird das Disposable bei nicht erfolgreichem Sammelvorgang also unbenutzt verworfen.

Bei derartigen integrierten oder hochintegrierten Messgeräten, insbesondere bei solchen für den mobilen Gebrauch wie beim Home-Monitoring, stellen sich somit verschiedene Probleme:
- Nach dem Stand der Technik wird in der Regel bei jeder Blutentnahme eine frische Lanzette verwendet. Daraus resultiert ein Lanzettenmagazin, das mehr Volumen in dem Blutanalysegerät beansprucht, als das Magazin für die Testelemente. Zur Verkleinerung des Blutanalysegerätes ist es wünschenswert, das Volumen des Lanzetten-Vorratsbehältnisses zu reduzieren.
- Im Stand der Technik wird davon ausgegangen, dass das integrierte Disposable, d.h. das kombinierte Stech- und Testelement zum einmaligen Gebrauch bestimmt ist und nach dem Abschluss eines Benutzungszykluses verworfen wird. Verworfen wird dann auch ein an sich ungebrauchter Test, wenn die Blutgewinnung nicht funktioniert hat. Für einen neuen Versuch muss ein neues Disposable verwendet werden.
- Für die Benutzer integrierter Disposables ist wegen der Kosten dieser Verbrauchsmaterialien, die höher sind als bei einfachen Stech- oder Testelementen, sehr wichtig, dass die Probenanalyse mit einer sehr hohen Erfolgsrate durchgeführt wird, so dass keine Disposables verschwendet werden. Diese Erfolgsrate muss deutlich über 90% liegen und in der Praxis nahezu 100% betragen, damit ein entsprechendes System von den Benutzern akzeptiert wird, insbesondere weil sie in zunehmendem Maß die Kosten für die Verbrauchsmaterialien selbst tragen müssen. Zur Erzielung einer hohen Erfolgsrate ist nach dem Stand der Technik ein großer technischer Aufwand erforderlich.

Aus der WO 2005/104949 A1 ist ein Analysegerät mit einem integrierten Stechgerät bekannt, bei dem mittels einer Kontrolleinrichtung geprüft wird, ob die nach dem Einstechen mit dem Stechelement an der Einstichstelle austretende Probe zum Durchführen einer Analyse mit einem Testelement ausreicht. Die Verwendung integrierter Analyseelemente mit Stech- und Testelement wird vorgeschlagen. Wenn die in einem Einstichvorgang an der Oberfläche der Haut angesammelte Probenmenge nicht zum Durchführen einer Analyse ausreicht, kann dies mittels einer Signaleinrichtung dem Benutzer mitgeteilt werden. Daraufhin wird das Gerät wieder über der Einstichwunde platziert und ein zweiter Einstichvorgang wird mit demselben oder einem anderen Stechelement durchgeführt. Zusätzliche Probemenge kann durch eine Expressionseinrichtung des Gerätes gewonnen werden, die zum Ausdrücken der Einstichstelle dient. Wenn nach dem ersten Einstichvorgang das Gerät versehentlich von der Einstichstelle abgenommen wurde, ist es gemäß dieser Druckschrift für den Benutzer auch möglich, die Haut manuell auszudrücken, um Flüssigkeit zu gewinnen, und das Gerät wieder über der Einstichstelle zu platzieren, um die Probe damit aufzunehmen und zu analysieren. Die Druckschrift beschreibt nur Fälle, in denen automatisch ein weiterer Einstich erfolgt, falls keine ausreichende Probenmenge gewonnen wurde. Allgemein wird bei Erkennung von unzureichender Probenmenge nach dem Motto verfahren: mehr von demselben, d.h. mehr Einstiche, mehr Stichtiefe, mehr Melkanstrengung etc., aber stets unter prinzipieller Beibehaltung des gerätetypischen Ablaufs wie beim ersten Versuch der Probengewinnung.

Ein vergleichbares Analysegerät wird in der US 2003/0208140 A1 vorgeschlagen. Auch dort wird überprüft, ob eine ausreichende Probenmenge gewonnen wurde. Es wird aber nicht erläutert, wie weiter verfahren wird, wenn festgestellt wurde, dass die Probenmenge nicht zum Durchführen einer Analyse ausreicht.

Das Gerät aus der WO 03/083469 A2 offenbart ein Stechgerät gemäß der Präambel des Anspruchs 1.

Der Erfindung liegt die Aufgabe zugrunde, ein Stechgerät bzw. ein Analysegerät zu schaffen, bei dem mit geringem technischem Aufwand eine sehr hohe Erfolgsrate beim Gewinnen einer Probe einer Körperflüssigkeit, insbesondere mit integrierten Disposables, erzielt wird.

Diese Aufgabe wird erfindungsgemäß durch ein Stechgerät bzw. ein Verfahren oder ein Analysegerät mit den Merkmalen der beigefügten unabhängigen Patentansprüche gelöst. Bevorzugte Ausgestaltungen und Weiterbildungen der Erfindung ergeben sich aus den abhängigen Patentansprüchen und der nachfolgenden Beschreibung mit zugehöriger Zeichnung.

Ein erfindungsgemäßes Stechgerät zum Erzeugen einer Einstichwunde in der Haut eines Menschen oder Tieres zum Gewinnen einer Probe einer Körperflüssigkeit mit einem ersten analytischen Verbrauchsmittel, das ein Stechelement umfasst, zum Durchführen einer Analyse eines medizinisch bedeutsamen Bestandteils der Probe mit einem zweiten analytischen Verbrauchsmittel, das ein Testelement umfasst, wobei das Stechgerät ein Andruckteil zum Anpressen des Stechgeräts an einen Körperteil, in dem mit dem Stechelement an einer Probenentnahmestelle eine Einstichwunde erzeugt werden soll, aus der eine Probe entnommen werden soll, einen Antrieb zum Antreiben eines in das Stechgerät eingesetzten Stechelements für eine Stechbewegung des Stechelements entlang eines vorbestimmten Einstechweges in eine Stechposition, wobei die Stechbewegung eine schnelle Einstichbewegung umfasst, auf die eine sich unmittelbar anschließende Rückzugsbewegung folgt, ein Gehäuse mit einer Körperteilanlageöffnung für den Körperteil, wobei das Stechelement in einer Ausgangsposition in dem Gehäuse angeordnet ist und an der Körperteilanlageöffnung mittels des Antriebs in den angelegten Körperteil in die Stechposition einstechbar ist, und eine Kontrolleinrichtung zum Überprüfen einer gewonnenen Probe darauf hin, ob die gewonnene Probenmenge ausreicht, um mit einem Testelement die Analyse durchzuführen, umfasst, weist also die Besonderheit auf, dass der Antrieb derart ausgebildet ist, dass in einem ersten Schritt eine Stechbewegung mit einem Stechelement zum Erzeugen einer Einstichwunde an einer Probenentnahmestelle für das Gewinnen einer Probe durchführbar ist, das Stechgerät derart ausgebildet ist, dass im Anschluss an die Stechbewegung mit der Kontrolleinrichtung überprüfbar ist, ob die gewonnene Probenmenge ausreicht, um mit einem Testelement die Analyse durchzuführen, und der Antrieb derart ausgebildet ist, dass in einem weiteren Schritt mindestens eines der analytischen Verbrauchsmittel in einer Ausfahrbewegung in eine Kontaktposition verfahrbar ist, in der es während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt bringbar ist, wenn gemäß dem Ergebnis der mit der Kontrolleinrichtung durchgeführten Überprüfung die bei der vorausgegangenen Stechbewegung gewonnene Probenmenge nicht zum Durchführen der Analyse ausreicht, wobei sich die Ausfahrbewegung von der Einstechbewegung dadurch unterscheidet, dass das analytische Verbrauchsmittel länger in der Kontaktposition als in der Stechposition verbleibt, so dass es in der Kontaktposition während der Ausfahrdauer von dem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt bringbar ist.

Der Antrieb ist also derart ausgebildet, dass mindestens eines der analytischen Verbrauchsmittel in eine Kontaktposition verfahrbar ist, in der es während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt bringbar ist, wenn gemäß dem Ergebnis einer mit der Kontrolleinrichtung durchgeführten Überprüfung der gewonnenen Probenmenge die bei einer vorausgegangenen Stechbewegung gewonnene Probenmenge nicht zum Durchführen der Analyse oder Diagnose ausreicht.

Es wird also nicht "mehr von demselben" versucht, sondern ein vollkommen anderer Modus eingenommen, in dem das Gerät nicht mehr wie ein integriertes, automatisches Messgerät verwendet wird, sondern wie ein herkömmliches, rein manuelles. Das Element, das im ersten Versuch die Probe automatisch hätte aufnehmen sollen, wird nun angeboten wie ein herkömmlicher Teststreifen, auf den die rein manuell ausgemolkene Probe rein manuell appliziert wird. Der erste Stechversuch dient dabei nur noch als Mittel, eine Einstichwunde zu erzeugen, als wäre es eine herkömmliche Stechhilfe. Der Versuch der automatischen Probengewinnung aus der Wunde war ja erfolglos.

Erst wenn sich beim manuellen Melken herausstellt, dass der Einstich nicht tief genug war, um auf blutführende Schichten zu stoßen, kann das selbe Testelement im automatischen Modus - nach Erhöhung der Stichtiefe - nochmals wie im Erstversuch automatisch verwendet werden. Das muss allerdings vom Anwender aktiv aufgerufen werden.

Durch ein erfindungsgemäßes Stechgerät mit einer Antriebseinheit, die zum einen den Einstichvorgang ausführen kann, sowie zum anderen einen Verfahrschritt des Disposables durchführt, wobei diese Funktionen auch durch zwei getrennte Antriebe realisiert werden können, und bei dem eine händige Blutaufgabe möglich ist, lassen sich die Nachteile der bekannten Geräte beheben, indem ein (erneutes) Wiederverwenden das Stechelements bzw. bei einem System für integrierte Disposables des integrierten Disposables ermöglicht wird, so dass entweder der Einstichvorgang wiederholt werden kann, oder das Disposable (erneut) aus dem System gefahren wird, so dass eine händige Blutaufgabe durch den Benutzer möglich ist, nachdem manuell durch das so genannte "Melken" des Fingers Blut gewonnen wurde.

Der erfindungsgemäße Bewegungsablauf unterscheidet sich vom Stand der Technik dadurch, dass mindestens eines der analytischen Verbrauchsmittel (Stechelement oder Testelement) mittels des Antriebs (des Stechelements oder des Testelements) in eine Kontaktposition verfahren wird, in der es nicht nur ganz kurz für den Einstich verbleibt, sondern während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt gebracht werden kann, wenn gemäß dem Ergebnis einer mit der Kontrolleinrichtung durchgeführten Überprüfung der gewonnenen Probenmenge die bei einer vorausgegangenen Stechbewegung gewonnene Probenmenge nicht zum Durchführen der Analyse oder Diagnose ausreicht.

Der Bewegungsablauf zum Verfahren des Verbrauchsmittels in die Kontaktposition, d.h. die Ausfahrbewegung, ist verschieden von der Stechbewegung zum Durchführung des Einstichs in der Stechposition, weil das Verbrauchsmittel zur manuellen Blutaufgabe in der Kontaktposition für einen gewissen Zeitraum verbleibt, im Gegensatz zur Stechbewegung, die durch ein schnelle Einstich- und Rückzugsbewegung gekennzeichnet ist. Unter Stechposition wird dabei die Position des Verbrauchsmittels zum Zeitpunkt der größten Einstichtiefe in die Probenentnahmestelle verstanden. Der Einfachheit halber können die Stechposition und die Kontaktposition sich jeweils auf die am weitesten auf die Probenentnahmestelle zu bewegte bzw. aus dem Stechgerät herausgefahrene Stelle des Verbrauchsmittels, insbesondere dessen Spitze beziehen. Natürlich kann in manchen Ausführungsformen vorgesehen sein, dass die gewählte Einstichtiefe und somit die Stechposition des Verbrauchsmittels vom Benutzer veränderbar einstellbar sind.

Der zeitliche Verlauf der Stech- und der Ausfahrbewegung sind ist jedoch verschieden, da in der Kontaktposition ein länger andauernder Stillstand des Verbrauchsmittels, d.h. eine Unterbrechung seiner Bewegung erfolgt, wogegen es in der Stechbewegung sofort nach dem Einstich aus der Stechposition zurückgezogen wird. Die Dauer des Zeitraum des Stillstands des Verbrauchsmittels in der Kontaktposition, während der das Verbrauchsmittel zum Durchführen bzw. Ermöglichen einer manuellen Blutaufgabe in der Kontaktposition verbleibt, kann in vorteilhaften Ausführungsformen länger als 0,5 sec, 1,0 sec, 2 sec, 3 sec, 4 sec, 5 sec, 7,5 sec, 10 sec, 12,5 sec, 15 sec oder 20 sec sein. Diese Zeit sollte so lange gewählt werden, dass sie für den Benutzer zum Durchführen der manuellen Blutaufgabe ausreicht. Das Verbrauchsmittel kann für eine fest vorgegebene Zeitdauer in der Kontaktposition verbleiben. In anderen Ausführungsformen kann aber auch das Verweilen in der Kontaktposition durch einen Befehl des Benutzers, wenn er die manuelle Probenaufnahme beendet hat, oder durch ein Signal der Kontrolleinrichtung, wenn diese das Aufnehmen einer ausreichenden Probenaufnahme erkannt hat, beendet werden.

Das Stechgerät wird nach dem ersten Einstichvorgang bewusst von der Einstichstelle abgenommen und es wird geprüft, ob die gewonnene Probenmenge zum Durchführen einer Analyse ausreicht. Falls die Probenmenge nicht ausreicht, wird die Einstichstelle dann gemolken und anschließend wird das Analysegerät an die gewonnene Probe herangeführt (oder umgekehrt, die gewonnene Probe auf der Haut wird an das Gerät herangeführt), so dass die Probenflüssigkeit vom analytischen Verbrauchsmittel aufgenommen wird, wieder an der Einstichstelle angesetzt, wobei sich das analytische Verbrauchsmittel in der Kontaktposition befindet und somit eine händige Blutaufgabe wie auf einen herkömmlichen Teststreifen erfolgen kann. Im Unterschied zum Stand der Technik fährt also das Stechgerät nicht automatisch mit der Probengewinnung, z.B. durch erneutes Einstechen mit derselben oder einem anderen Stechelement) fort, sondern das Stech- bzw. Analysegerät wird von der Einstichstelle weggenommen und auf einen manuellen Modus zum händigen Aufnehmen der Probe mit dem Testelement umgeschaltet.

Ein Unterschied zum Stand der Technik ist darin zu sehen, dass bei der Erfindung von einem Normalbetriebmodus in einen alternativen Ablauf mit manueller Blutaufnahme umgeschaltet wird, wenn eine Unterdosierung festgestellt wird. Es wird also zunächst ein Einstich durchgeführt, im Anschluss daran geprüft, ob die gewonnene Probenmenge ausreicht, und falls dies nicht der Fall sein sollte, wechselt das Stechgerät in einen manuellen Modus, bei dem durch Melken der Einstichstelle von Hand zusätzliches Blut gewonnen wird und dann ein händiger Auftrag auf das zwischenzeitlich automatisch oder auf einen manuellen Befehl des Benutzers hin von dem Stechgerät in eine hierzu geeignete Position verfahrene Testelement erfolgt. Im Unterschied zum Stand der Technik wird also für den Fall, dass die Prüfung ergibt, dass keine ausreichende Probenmenge gewonnen wurde, die Messung weder verworfen noch erfolgt unmittelbar ein erneuter Einstich, sondern das Stechgerät wechselt in den manuellen Modus zum händigen Auftrag des durch Melken gewonnen Bluts auf das Testelement.

Zum Erleichtern der manuellen Blutaufnahme ist es bevorzugt, wenn das mindestens eine analytische Verbrauchsmittel in dem weiteren Schritt während der Ausfahrdauer in der Kontaktposition aus einer Austrittsöffnung des Stechgerätes herausragt, vorzugsweise weit herausragt, damit der Benutzer die Probenentnahmestelle gut einsehen und das Stechgerät mit dem herausragenden analytischen Verbrauchsmittel an die Probenentnahmestelle heranführen und deren Kontakt mit dem herausgefahrenen analytischen Verbrauchsmittel durch ein händiges Hinführen des Stechgerätes herstellen kann.

Nach einem weiteren vorteilhaften Merkmal kann vorgesehen sein, dass die Kontaktposition des analytischen Verbrauchsmittels in dem weiteren Schritt der Ausfahrbewegung verschieden zu der Stechposition ist, die es in dem ersten Schritt der Stechbewegung einnimmt. Im allgemeinen ist es zwar möglich, dass die Kontaktposition und die Stechposition übereinstimmen, also das analytische Verbrauchsmittel in der Ausfahrbewegung genauso weit wie in der Stechwegung in Richtung auf die Probenentnahmestelle bewegt bzw. aus dem Stechgerät herausgefahren wird. In vorteilhaften Ausführungsformen wird das analytische Verbraüchsmittel jedoch in der Ausfahrbewegung weiter auf die Probenentnahmestelle zu bewegt bzw. aus dem Stechgerät herausgefahren werden als in der Stechbewegung, d.h. die Kontaktposition liegt weiter in auswärtiger Richtung des Stechgeräts als die Stechposition, damit der Benutzer die Probenentnahmestelle gut einsehen und das Stechgerät mit dem analytischen Verbrauchsmittel an die Probenentnahmestelle heranführen und deren Kontakt mit dem analytischen Verbrauchsmittel durch ein händiges Hinführen des Stechgerätes herstellen kann.

In bevorzugten Ausführungsformen ist das mindestens eine analytische Verbrauchsmittel, das in dem weiteren Schritt in die Kontaktposition verfahren wird, ein analytisches Verbrauchsmittel, das ein Stechelement oder ein Testelement oder ein integriertes Disposable, das sowohl ein Stechelement zum Durchführen des Einstichvorgangs für das Gewinnen der Probe einer Körperflüssigkeit als auch ein Testelement zum Durchführen der Analyse eines medizinisch bedeutsamen Bestandteils der Probe umfasst, umfasst.

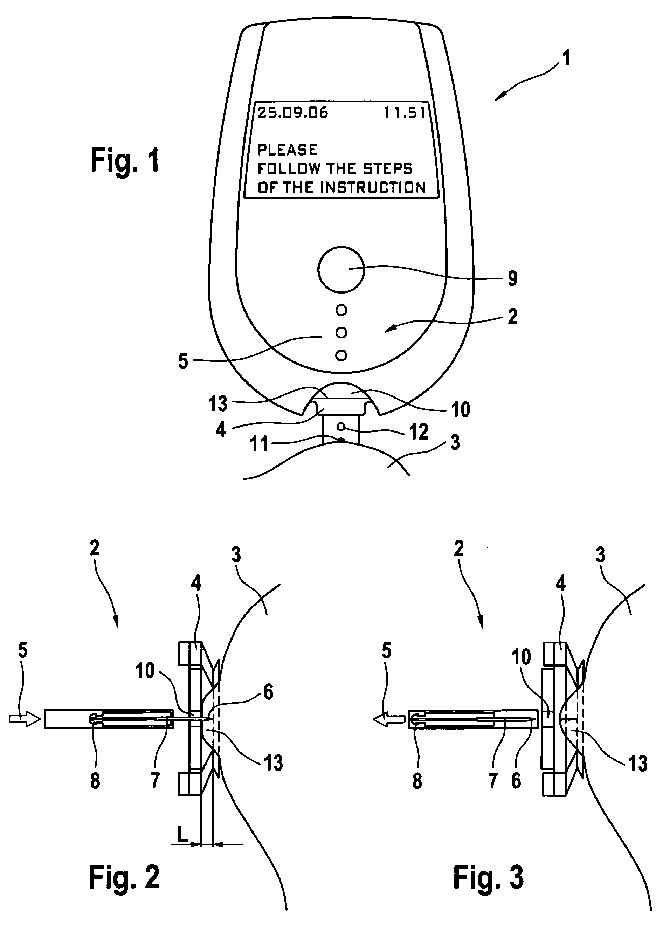

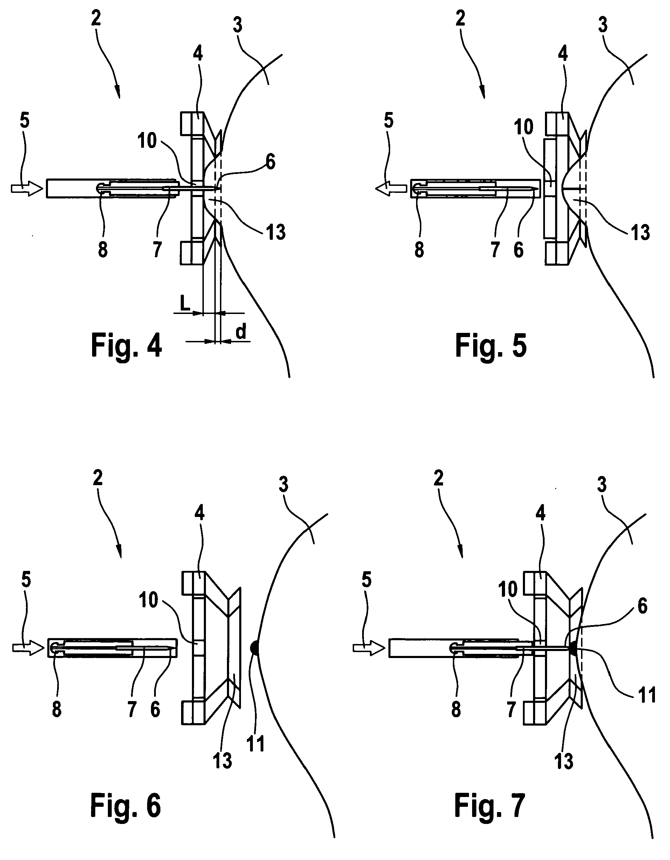

Der normale Ablauf ist in den Figuren 2 bis 5 und der alternative, bei Unterdosierung durchgeführte Ablauf in den Figuren 6 und 7 der Patentanmeldung dargestellt. Ein erfindungsgemäßes Stech- bzw. Analysegerät umfasst also vorzugsweise einen üblichen Betriebsmodus (Normalbetriebmodus) gemäß dem Stand der Technik sowie einen zusätzlichen Sonderzustand (manueller Modus), bei dem das Testelement - vorzugsweise weit - aus dem Analysegerät herausgefahren wird, um nach einer zuvor festgestellten zu geringen Probenmenge, die in einem vorausgehenden Einstichvorgang gewonnen wurde, mittels eines zwischenzeitlich durchgeführten Knetens der Einstichstelle zu versuchen, eine zusätzliche Probenmenge zu gewinnen und diese durch ein manuelles Bewegen des Stechgerätes in Kontakt mit dem Testelement zu bringen. Das Stechgerät umfasst demnach vorzugsweise einen automatischen Betriebsmodus, in dem der erste Schritt einer Stechbewegung mit einem Stechelement zum Erzeugen einer Einstichwunde an einer Probenentnahmestelle für das Gewinnen einer Probe und im Anschluss an die Stechbewegung mit der Kontrolleinrichtung die Überprüfung durchführbar ist, ob die gewonnene Probenmenge ausreicht, um mit einem Testelement die Analyse durchzuführen, und weist einen manuellen Betriebsmodus auf, in dem automatisch oder durch einen Auslösebefehl des Benutzers mittels des Antriebs in dem weiteren Schritt mindestens eines der analytischen Verbrauchsmittel in die Kontaktposition verfahrbar ist, in der es während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt bringbar ist, wenn die gemäß dem Ergebnis der mit der Kontrolleinrichtung durchgeführten Überprüfung die bei der vorausgegangenen Stechbewegung gewonnene Probenmenge nicht zum Durchführen der Analyse ausreicht.

Von dem aus der WO 2005/104949 A1 bekannten Stechgerät unterscheidet sich das erfindungsgemäße Stechgerät dadurch, dass nach dem Stand der Technik nach einem erfolglosen Blutsammelprozess das Testelement zur Blutaufnahme direkt verwendet wird, wobei das Testelement in der gleichen Probenaufnahmestellung ist, die es auch beim automatischen Blutsammelvorgang einnimmt. Im Gegensatz dazu kann bei dem erfindungsgemäßen Stechgerät ein Blutsammelvorgang durch ein Stechelement erfolgen, das zunächst einen Einstichvorgang ausführt. Sofern eine Probenaufnahme durch das Stechelement selbst erfolgt, erfordert dies einen Antrieb, der das Stechelement in eine Position verfahren muss, die verschieden ist vom Bewegungsablauf des Einstichvorgangs. Insbesondere ist es hierfür erforderlich, dass bei einer manuellen Probenaufnahme das Stechelement in seiner Position zur Probenaufnahme, d.h. in der Kontaktposition, für einen längeren Zeitraum verbleiben muss, so dass es in dieser Kontaktposition von einem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt bringbar ist. Das aus dem Stand der Technik bekannte Stechgerät weist dagegen keinen Antrieb auf, der das analytische Verbrauchsmittel, insbesondere ein Verbrauchsmittel, das ein Testelement umfasst, in eine Probenaufnahmeposition verfährt, die sich von den Positionen des Verbrauchsmittels während des automatischen Analysevorgangs mittels des Systems unterscheidet.

Die Ausfahrdauer des analytischen Verbrauchsmittels aus dem Gerät in die Kontaktposition im Bereich der Körperteilanlageöffnung kann fest vorgegeben sein, beispielsweise mehrere Sekunden bis einige Minuten, nach deren Ablauf das analytische Verbrauchsmittel automatisch von dem Antrieb in das Gerät zurückgezogen oder ausgeworfen wird. Das Ende der Ausfahrdauer kann dabei dem Benutzer durch vorausgehende Signale angekündigt werden. In anderen Ausführungsformen kann die Ausfahrdauer aber auch erst durch eine Betätigung eines Bedienelements durch den Benutzer beendet werden.

Bei dem in der Kontaktposition des analytischen Verbrauchsmittels manuell von dem Benutzer hergestellten Kontakt zwischen Körperteil bzw. Probenflüssigkeit und dem analytischen Verbrauchsmittel, das die Körperflüssigkeit aufnimmt, kann der Körperteil in derselben Position wie bei dem Einstechen an der Körperteilanlageöffnung des Gerätes anliegen oder sich vorzugsweise auch in einem Abstand dazu befinden, damit der Benutzer die Probenentnahmestelle besser einsehen und den Kontakt herstellen kann.

Mit der erfindungsgemäßen Erfolgskontrolle ist es also möglich, für den Fall, dass das erste Stechen nicht zu einer erfolgreichen Blutentnahme geführt hat, dies zu erkennen und die zuvor gestochene Fingerbeere, die noch die Einstichwunde aufweist, zu kneten und zu massieren ("melken"), um die Durchblutung zu fördern und dadurch einen Blutaustritt zu erzielen. Das auf diese Weise gewonnene Blut wird dann händig durch den Benutzer an der Auftragstelle des analytischen Verbrauchsmittels zur Analyse aufgegeben.

Diese Vorgehensweise ist in der Regel sogar vorteilhafter als ein zweites Stechen, weil ein zweites Stechen die Erfolgsquote für das Gewinnen einer ausreichenden Blutmengenprobe nicht unbedingt erhöhen würde, beispielsweise weil an der zuvor gestochenen Stelle wegen einer geringen Durchblutung wenig Blut vorhanden ist. Das Erkennen eines beim ersten Stechen auftretenden Fehlers und das nachfolgende händige Auftragen der Blutprobe mit vorausgehendem Kneten der Einstichstelle weist den in der Praxis sehr großen Vorteil auf, dass auch bei ungünstigen Bedingungen der Probeentnahmestelle mit geringem technischen Aufwand eine sehr hohe Erfolgsrate bei der Verwendung eines Stechelements, insbesondere eines integrierten Disposables, von über 95% und somit eine große Akzeptanz für den Benutzer erzielt werden kann.

Gemäß einer anderen Ausführungsform ist es aber auch möglich, dass nach dem ersten Stich mit einem Stechelement eine Kontrolle des Blutgewinnungserfolges zwischengeschaltet wird, wonach ggf. die Stecheinrichtung erneut in Bereitschaft versetzt wird, damit der Anwender u.U. nach einer Korrektur der Stechtiefeneinstellung erneut einen Stich ausführen kann, ohne das Stechelement oder Disposable vorher austauschen zu müssen.

Nach einem ersten vorteilhaften Merkmal kann vorgesehen sein, dass die Kontrolleinrichtung zum visuellen Kontrollieren der Probenmenge durch den Benutzer des Gerätes ausgebildet ist. Die Kontrolle der ausreichenden Probenaufnahme kann beispielsweise im Falle von Systemen mit Testelementen mit integrierter Lanzette durch den Anwender visuell erfolgen. Da keine automatische Blutgewinnung erfolgt, sondern von Hand gemolken werden muss, fällt das besonders leicht. Je nach Ausführungsform des Gerätes kann einfach erneut der Stechapparat gespannt werden oder ein Menüpunkt zur Stichwiederholung wird aufgerufen, wonach der Stechantrieb wieder in Bereitschaft tritt. Diese Funktion kann im Anwendungsgerät (Stechgerät oder Analysegerät) vorgesehen werden, was je nach Funktionsweise leicht möglich ist.

In anderen Ausführungsformen kann auch vorgesehen sein, dass die Kontrolleinrichtung eine automatische Probenmengenerkennungseinrichtung oder Unterdosiererkennungseinrichtung umfasst. Bei einer automatischen Bluterkennung, gemäß der festgestellt wird, dass nicht ausreichend Blut auf das Testfeld gelangt ist, kann automatisch das Disposable zur händigen Blutaufgabe verfahren werden. Eine automatische Bluterkennung bzw. Unterdosierungserkennung ist im Stand der Technik für Teststreifen hinreichend bekannt. Beispielsweise beschreibt das Dokument US 6,055,060 eine Unterdosierungserkennung. Vorteilhafterweise ist darüber hinaus das System in der Lage, zunächst einen wiederholten Einstichvorgang auszuführen, bevor eine händige Blutaufgabe durch den Benutzer möglich ist.

Bei Systemen mit automatischer Blutgewinnung und ebenso automatischem Transfer der gewonnenen Probe auf die Testchemie kann beispielsweise die Benetzungskontrolle der Testchemie bzw. die Testauswertung dazu herangezogen werden, den Sammelerfolg festzustellen und ggf. automatisch mit demselben Stechelement bzw. Disposable erneut Stichbereitschaft herzustellen. Hierbei eignen sich kurvengesteuerte Systeme nach dem OWADAC-Prinzip (OWADAC= one ay alternating drive and cocking) besonders gut, aber auch ballistische Systeme lassen sich ohne großen Aufwand so gestalten, dass ohne Austausch des Stechelements bzw. Disposables der Startzustand wiederhergestellt werden kann.

Selbst wenn im Falle von Samplern der Erfolg der Blutgewinnung erst bei der Übertragung auf die Testchemie festgestellt werden kann, kann in den meisten Ausführungsformen von Disposables (Stechelemente oder integrierte Disposables) und Analysegeräten die Testsequenz um einige Schritte zurückgefahren werden, so dass mit dem selben Disposable ein neuer Versuch gestartet werden kann. Sogar bei vielen Systemen mit Magazinierung von Testelementen steht einem zweiten Versuch der Blutgewinnung mit demselben Disposable nichts im Wege. Es ist lediglich darauf zu achten, dass für die verschiedenen Ablaufschritte keine irreversiblen Vorgänge herangezogen werden wie z.B. das Weiterspulen von Bändern, wenn aus Platz- und Kostengründen keine Rückspulfunktion vorgesehen ist, oder das Umbiegen von Blechteilen für die Blutübertragung wie bei einigen Varianten von Samplern.

Im Zusammenhang von "GAM" (= get & measure) mit den Disposables, bei dem Stechen und Messen vollständig in ein Gerät integriert ist, ergibt sich zusätzlich noch eine Optimierung, die den Erfolg für den Anwender, zu einem Analysewert, beispielsweise einem Blutglukosewert zu gelangen, noch erhöht.

Die Kontrolleinrichtung kann eine Signaleinheit zum Signalisieren einer für die Durchführung der Analyse oder Diagnose zu geringen Probenmenge an den Benutzer umfassen. Die Signaleinrichtung kann ein optisches, akustisches, haptisches oder sonstiges Signal abgeben. Durch diese kann dem Benutzer beispielsweise signalisiert werden, dass er sich entscheiden muss, ob er mit dem Stechelement bzw. dem integrierten Disposable einen weiteren Einstichversuch an derselben oder einer anderen Probenentnahmestelle durchführen möchte oder lieber einen händigen Probenauftragsschritt durchführen möchte, wobei der Benutzer seine Entscheidung durch Betätigen eines Bedienelement eingeben kann. Demgemäß kann es vorteilhaft sein, wenn das Stechelement zum Durchführen eines weiteren Einstichvorgangs mit dem Stechelement in die Ausgangs- oder eine Bereitschaftsposition verfahrbar ist, wenn bei der Verwendung des Stechelements zum Entnehmen einer Probe erstmals, d.h. nach dem ersten Schritt einer Stechbewegung mit einem Stechelement und der Überprüfung, ob die dabei gewonnene Probenmenge ausreicht, eine für die Durchführung der Analyse oder Diagnose zu geringe Probenmenge erkannt wird, und der Antrieb derart ausgebildet ist, dass nach dem Durchführen des weiteren Einstichvorgangs in dem weiteren Schritt mindestens eines der analytischen Verbrauchsmittel in eine Kontaktposition verfahrbar ist, in der es während einer Ausfahrdauer im Bereich der Körperteilanlageöffnung in einer Position verbleibt, in der es von einem Benutzer des Stechgerätes durch eine manuelle Positionierung des Stechgerätes zum manuellen Aufnehmen einer Probe, die durch manuelles Melken der Probenentnahmestelle im Bereich der Einstichwunde gewonnen wurde, mit der Probe in Kontakt bringbar ist, wenn die gemäß dem Ergebnis der mit der Kontrolleinrichtung durchgeführten Überprüfung die bei dem vorausgegangenen weiteren Einstichvorgang gewonnene Probenmenge nicht zum Durchführen der Analyse ausreicht. Der weitere Einstichvorgang kann vorteilhafterweise mit einer vergrößerten Einstichtiefe durchführbar sein. In anderen Ausführungsformen kann dem Benutzer durch die Signaleinrichtung auch direkt signalisiert werden, dass als nächstes eine händige Probenaufgabe mit dem Stechelement bzw. integrierten Disposable durchzuführen ist.

Allgemein kann es vorteilhaft sein, wenn das Stechelement in die Kontaktposition verfahrbar ist, wenn bei der Verwendung des Stechelements zum Entnehmen einer Probe erstmals, d.h. nach dem ersten Schritt einer Stechbewegung mit einem Stechelement und der Überprüfung, ob die dabei gewonnene Probenmenge ausreicht, eine für die Durchführung der Analyse oder Diagnose zu geringe Probenmenge erkannt wird. Vorteilhafte Ausführungsformen in Verbindung mit einer automatischen Kontrolleinrichtung können darin bestehen, dass das Stechelement automatisch in die Kontaktposition verfahren wird, wenn die Kontrolleinrichtung eine für die Durchführung der Analyse oder Diagnose zu geringe Probenmenge erkennt.

Weitere Einzelheiten und Vorteile der Erfindung werden anhand eines Ausführungsbeispiels unter Bezugnahme auf die beigefügten Zeichnungen erläutert. Die dabei beschriebenen Merkmale können einzeln und in Kombination verwendet werden, um bevorzugte Ausgestaltungen der Erfindung zu schaffen. Gleiche oder einander entsprechende Bauteile sind dabei mit übereinstimmenden Bezugszeichen gekennzeichnet.

Es zeigen:
- Fig. 1: eine Ansicht eines Analysegerätes mit einem integriertem erfindungsgemäßen Stechgerät an einem Körperteil;
- Fig. 2: einen ersten Einstichvorgang mit einem Stechgerät;
- Fig. 3: einen Rückzugsvorgang zu Fig. 2;
- Fig. 4: einen zweiten Einstichvorgang mit vergrößerter Einstichtiefe;
- Fig. 5: einen Rückzugsvorgang Fig. 4;
- Fig. 6: einen ersten Teilschritt eines manuellen Blutauftrags auf ein Stechelement; und
- Fig. 7: einen zweiten Teilschritt zu Fig. 6.

Die Fig. 1 zeigt eine Ansicht eines Ausführungsbeispiels eines Analysegeräts 1 mit einem integrierten Stechgerät 2 zum Erzeugen einer Einstichwunde in einer Fingerkuppe 3 zum Gewinnen und Analysieren einer Blutprobe aus der Fingerkuppe 3. Hierzu umfasst es ein Andruckteil 4 zum Anpressen an die Fingerkuppe 3, aus der die Probe entnommen werden soll, und einen Antrieb 5 zum Antreiben eines Stechelements 6 für eine Stechbewegung. Das Andruckteil 4 ist als Andruckkonus ausgebildet, der in der angepressten Fingerkuppe 3 einen erhöhten Körperflüssigkeitsdruck erzeugt und somit den Austritt von Körperflüssigkeit aus einer mit einem in das Stechgerät 2 eingesetzten Stechelement 6 erzeugten Einstichwunde begünstigt.

Bei dem in Fig. 1 schematisch dargestellten Analysegerät 1 handelt es sich um ein integriertes System zur Gewinnung und Analyse einer Körperflüssigkeitsprobe. Das Stechelement 6 enthält deshalb einen Kapillarkanal 7, der in eine Analysezone 8 mündet, die mit Testchemikalien behandelt ist und deshalb eine von der Analytkonzentration abhängige Farbänderung erfährt.

Zur Analyse wird die Analysezone 8 mit einer Lichtquelle bestrahlt und reflektierte Strahlung von einem Detektor detektiert. Die Lichtquelle wird von einer Steuereinheit gesteuert und das Signal des Detektors von einer Auswerteeinheit ausgewertet. Vorzugsweise regelt die Auswerteeinheit auch die Steuereinheit. Die Auswerteeinheit führte eine Auswertung des Detektorsignals durch, um die Konzentration des in der Probe der Körperflüssigkeit enthaltenen Analyten zu ermitteln. Das Analyseergebnis wird mit einer Ausgabeeinheit, beispielsweise einer Flüssigkristallanzeige ausgegeben.

Einen besonders hohen Benutzerkomfort bieten somit Stechgeräte 2, mit denen nicht nur eine Einstichwunde erzeugt wird, sondern die zusätzlich eine Messeinrichtung zum Untersuchen einer gewonnenen Körpertlüssigkeitsprobe umfassen. Geeignete Messeinrichtungen, beispielsweise zur photometrischen oder elektrochemischen Untersuchung, sind in handelsüblichen Analysehandgeräten 1 zum Bestimmen des Blutglukosegehalts enthalten. Beispielsweise können eine Probenaufnahmeeinheit und ein Testfeld zur elektrochemischen oder photometrischen Bestimmung einer Analytkonzentration in das Stechelement 6 integriert sein. Geeignete Stechelemente, die einen Kapillarkanal aufweisen, in den Körperflüssigkeit während einer Sammelphase eindringen und zu dem Testfeld gelangen kann, sind im Stand der Technik, beispielsweise aus der US 2003/0171699 A1, bekannt. Bei derartigen Stechgeräten macht die Probenaufnahme für den Benutzer keine zusätzlichen Handhabungsschritte erforderlich. Dies ist insbesondere für Benutzer, deren motorische Beweglichkeit durch Alter oder Krankheit eingeschränkt ist, ein wichtiger Vorteil.

Das dargestellte Analysegerät 1 weist ein Auslösemittel auf, durch dessen Betätigung ein Benutzer nach dem Anpressen oder durch Anpressen des Andruckteils 4 eine Einstichbewegung auslösen kann. Bei dem dargestellten Ausführungsbeispiel ist das Auslösemittel als Knopf 9 ausgebildet. Dabei kann auch eine Sicherungseinrichtung vorgesehen sein, mit der das Stechgerät 2 aus einem Sperrzustand in einem Auslösezustand versetzt wird.

Die Figuren 2 bis 7 zeigen die für die erfindungsgemäße Funktion wesentlichen Komponenten des Stechgeräts 2 in dem Analysegerät 1 von Fig. 1. Die Figuren 2 und 3 zeigen einen ersten Schritt, nämlich in Fig. 2 einen Einstichvorgang mit dem Stechgerät 2 mit einer Einstechtiefe L von beispielsweise 1,0 mm. Die Fingerkuppe 3 wird zum Durchführen des Einstichs in der Körperteilanlageöffnung 13 an das Gerät 1 bzw. das Andruckteil 4 angepresst. Die Fig. 3 zeigt die Position des Stechelements 6 nach seinem vollständigen Zurückziehen in das Stechgerät 2, und in dieser Position des Stechelements 6 wird visuell oder automatisch geprüft, ob bei dem Einstichvorgang gemäß Fig. 2 eine ausreichende Probenmenge gewonnen wurde.

Ist dies nicht der Fall, kann der in den Figuren 4 und 5 dargestellte zweite Schritt eines weiteren Einstichvorgangs manuell oder automatisch ausgelöst werden. Dabei zeigt Fig. 4 einen Einstichvorgang, der mit einer erhöhten Einstechtiefe, beispielsweise mit einer Einstechtiefenzugabe d von 0,5 mm durchgeführt werden kann, und Fig. 5 zeigt das Stechelement 6 in derselben Position wie in Fig. 3, wobei wiederum geprüft wird, ob eine ausreichende Menge einer Probe gewonnen wurde.

Wenn dies nicht der Fall ist, erfolgt der in den Figuren 6 und 7 dargestellte dritte Schritt, wobei dieser Schritt auch unmittelbar nach dem ersten Schritt (Figuren 2 und 3) unter Auslassung des zweiten Schrittes (Figuren 4 und 5) durchgeführt werden kann, wenn gemäß Fig. 3 festgestellt wird, dass keine ausreichende Probenmenge gewonnen wurde. Die Fig. 6 zeigt das Stechelement 6 noch in der in das Stechgerät 2 zurückgezogenen Ausgangsposition entsprechend zu den Figuren 2 und 4, aus der es gemäß Fig. 7 in eine Kontaktposition verfahren wird, in der es aus der Austrittsöffnung 10 des Stechgerätes 2 im Bereich der Körperteilanlageöffnung 13 derart während einer gewissen Ausfahrdauer herausragt, dass es von einem Benutzer des Gerätes durch eine manuelle Positionierung des Stechgerätes 2 mit der Probenentnahmestelle bzw. mit einer durch manuelles Melken im Bereich der Einstichstelle gewonnenen Probe in Kontakt gebracht werden kann. Dabei wird der Benutzer vorteilhafterweise das Gebiet um die Probenentnahmestelle zuvor kneten oder massieren, um das Austreten eines Bluttropfens 11 zu fördern, der dann von der Spitze des aus dem Stechgerät 2 mittels des Antriebs herausgefahrenen Stechelements 6 in Kontakt gebracht werden kann, wodurch mit dem Stechelement 6 die Probe aus dem Bluttropfen 11 entnommen wird.

Das manuelle Aufnehmen eines Bluttropfens 11 mit dem Stechelement 6 kann durch eine schrittweise Benutzerführung, die in einer Anzeige des Analysegerätes 1 erscheint, geführt werden. Zum Erleichtern der manuellen Aufnahme der Probe mit dem Stechgerät 2 kann es vorteilhaft sein, wenn die Probenaufnahmestelle mittels einer in das Stechgerät 2 bzw. das Analysegerät 1 integrierten Beleuchtung beleuchtet wird. Die Fig. 1 zeigt diese Beleuchtung 12 in einer Position des Analysegeräts 1 gemäß Fig. 6.

In abgewandelten Ausführungsformen kann die manuelle Probenaufnahme gemäß dem dritten Schritt auch dadurch erfolgen, dass nicht das Stechelement 6, sondern das Testelement, mit dem die Analyse durchgeführt wird, entweder einem Disposable oder einem integrierten Disposable, mit der von der Probenentnahmestelle aufzunehmenden, sich im Bereich der Körperteilanlageöffnung 13 befindenden Probe manuell durch eine entsprechende händige Positionierung des Analysegerätes 1 in Kontakt gebracht wird.

### Bezugszeichenliste

- 1: Analysegerät
- 2: Stechgerät
- 3: Fingerkuppe
- 4: Andruckteil
- 5: Antrieb
- 6: Stechelement
- 7: Kapillarkanal
- 8: Analysezone
- 9: Knopf
- 10: Austrittsöffnung
- 11: Bluttropfen
- 12: Beleuchtung
- 13: Körperteilanlageöffnung
- L: Einstechtiefe
- d: Einstechtiefenzugabe

## Patentansprüche

1. Stechgerät (2) zum Erzeugen einer Einstichwunde in der Haut eines Menschen oder Tieres zum Gewinnen einer Probe einer Körperflüssigkeit mit einem ersten analytischen Verbrauchsmittel, das ein Stechelement (6) umfasst, zum Durchführen einer Analyse eines medizinisch bedeutsamen Bestandteils der Probe mit einem zweiten analytischen Verbrauchsmittel, das ein Testelement umfasst, wobei das Stechgerät (2)
ein Andruckteil (4) zum Anpressen des Stechgeräts (2) an einen Körperteil, in dem mit dem Stechelement (6) an einer Probenentnahmestelle eine Einstichwunde erzeugt werden soll, aus der eine Probe entnommen werden soll,
einen Antrieb (5) zum Antreiben eines in das Stechgerät (2) eingesetzten Stechelements (6) für eine Stechbewegung des Stechelements (6) entlang eines vorbestimmten Einstechweges in eine Stechposition, wobei die Stechbewegung eine schnelle Einstichbewegung umfasst, auf die eine sich unmittelbar anschließende Rückzugsbewegung folgt,
ein Gehäuse mit einer Körperteilanlageöffnung (13) für den Körperteil, wobei das Stechelement (6) in einer Ausgangsposition in dem Gehäuse angeordnet ist und an der Körperteilanlageöffnung (13) mittels des Antriebs (5) in den angelegten Körperteil in die Stechposition einstechbar ist, und
eine Kontrolleinrichtung zum Überprüfen einer gewonnenen Probe darauf hin, ob die gewonnene Probenmenge ausreicht, um mit einem Testelement die Analyse durchzuführen, umfasst,
der Antrieb (5) derart ausgebildet ist, dass in einem ersten Schritt eine Stechbewegung mit einem Stechelement (6) zum Erzeugen einer Einstichwunde an einer Probenentnahmestelle für das Gewinnen einer Probe durchführbar ist,
das Stechgerät (2) derart ausgebildet ist, dass die in dem ersten Schritt gewonnene Probe mit dem ersten analytischen Verbrauchsmittel aufnehmbar ist, indem die gewonnene Probe mit dem ersten analytischen Verbrauchsmittel in Kontakt gebracht wird,
das Stechgerät (2) derart ausgebildet ist, dass im Anschluss an die Stechbewegung mit der Kontrolleinrichtung überprüfbar ist, ob die gewonnene, aufgenommene Probenmenge ausreicht, um mit einem Testelement die Analyse durchzuführen,
wobei das Stechgerät (2) derart ausgebildet ist, dass die gewonnene, aufgenommene Probenmenge zum Durchführen einer Analyse zu einem Testelement transportierbar ist, **dadurch gekennzeichnet, dass**
der Antrieb (5) derart ausgebildet ist, dass in einem weiteren Schritt mindestens eines der analytischen Verbrauchsmittel in einer Ausfahrbewegung in eine Kontaktposition verfahrbar ist, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe, die durch manuelles Melken der Probenentnahmestelle im Bereich der Einstichwunde gewonnen wurde, mit der Probe in Kontakt bringbar ist, wenn gemäß dem Ergebnis der mit der Kontrolleinrichtung durchgeführten Überprüfung die bei der vorausgegangenen Stechbewegung gewonnene Probenmenge nicht zum Durchführen der Analyse ausreicht,
wobei sich die Ausfahrbewegung von der Einstechbewegung dadurch unterscheidet, dass das analytische Verbrauchsmittel länger in der Kontaktposition als in der Stechposition verbleibt, so dass es in der Kontaktposition während der Ausfahrdauer von dem Benutzer des Stechgerätes (2) durch eine manuelle Positionierung des Stechgerätes (2) zum manuellen Aufnehmen einer Probe, die durch manuelles Melken der Probenentnahmestelle im Bereich der Einstichwunde gewonnen wurde, mit der Probe in Kontakt bringbar ist.

2. Stechgerät (2) nach Anspruch 1, **dadurch gekennzeichnet, dass** das mindestens eine analytische Verbrauchsmittel in dem weiteren Schritt während der Ausfahrdauer in der Kontaktposition aus einer Austrittsöffnung (10) des Stechgerätes (2) herausragt.

3. Stechgerät (2) nach einem der vorhergehenden Ansprüche, **dadurch gekennzeichnet, dass** die Kontaktposition des analytischen Verbrauchsmittels in dem weiteren Schritt der Ausfahrbewegung verschieden zu der Stechposition ist, die es in dem ersten Schritt der Stechbewegung einnimmt.

4. Stechgerät (2) nach dem vorhergehenden Anspruch, **dadurch gekennzeichnet, dass** der Antrieb (5) derart ausgebildet ist, dass das analytische Verbrauchsmittel in der Ausfahrbewegung weiter auf die Probenentnahmestelle zu bewegt bzw. aus dem Stechgerät (2) herausgefahren wird als in der Stechbewegung.

5. Stechgerät (2) nach einem der vorhergehenden Ansprüche, **dadurch gekennzeichnet, dass** das mindestens eine analytische Verbrauchsmittel, das in dem weiteren Schritt in die Kontaktposition verfahren wird, ein analytisches Verbrauchsmittel ist, das ein Stechelement (6) oder ein Testelement oder ein integriertes Disposable, das sowohl ein Stechelement (6) zum Durchführen des Einstichvorgangs für das Gewinnen der Probe einer Körperflüssigkeit als auch ein Testelement zum Durchführen der Analyse eines medizinisch bedeutsamen Bestandteils der Probe umfasst, umfasst.

6. Stechgerät nach einem der vorhergehenden Ansprüche, **dadurch gekennzeichnet, dass** das erste und das zweite analytische Verbrauchsmittel in einem analytischen Verbrauchsmittel integriert sind, das ein integriertes Disposable ist, das sowohl ein Stechelement (6) zum Durchführen des Einstichvorgangs für das Gewinnen der Probe einer Körperflüssigkeit als auch ein Testelement zum Durchführen der Analyse eines medizinisch bedeutsamen Bestandteils der Probe umfasst.

7. Stechgerät (2) nach einem der vorhergehenden Ansprüche, **dadurch gekennzeichnet, dass** die Kontrolleinrichtung zum visuellen Kontrollieren der Probenmenge durch den Benutzer des Gerätes ausgebildet ist.

8. Stechgerät (2) nach einem der vorhergehenden Ansprüche, **dadurch gekennzeichnet, dass** die Kontrolleinrichtung eine automatische Probenmengenerkennungseinrichtung oder Unterdosiererkennungseinrichtung umfasst.

9. Stechgerät (2) nach einem der vorhergehenden Ansprüche, **dadurch gekennzeichnet, dass** es einen automatischen Betriebsmodus umfasst, in dem der erste Schritt einer Stechbewegung mit einem Stechelement (6) zum Erzeugen einer Einstichwunde an einer Probenentnahmestelle für das Gewinnen einer Probe und im Anschluss an die Stechbewegung mit der Kontrolleinrichtung die Überprüfung durchführbar ist, ob die gewonnene, aufgenommene Probenmenge ausreicht, um mit einem Testelement die Analyse durchzuführen, und einen manuellen Betriebsmodus aufweist, in dem automatisch oder durch einen Auslösebefehl des Benutzers mittels des Antriebs (5) in dem weiteren Schritt mindestens eines der analytischen Verbrauchsmittel in die Kontaktposition verfahrbar ist, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe, die durch manuelles Melken der Probenentnahmestelle im Bereich der Einstichwunde gewonnen wurde, mit der Probe in Kontakt bringbar ist, wenn die gemäß dem Ergebnis der mit der Kontrolleinrichtung durchgeführten Überprüfung die bei der vorausgegangenen Stechbewegung gewonnene Probenmenge nicht zum Durchführen der Analyse ausreicht.

10. Stechgerät (2) nach einem der vorhergehenden Ansprüche, **dadurch gekennzeichnet, dass** das mindestens eine analytische Verbrauchsmittel des Antriebs (5) in die Kontaktposition verfahrbar ist, wenn bei der Verwendung des Stechelements (6) zum Entnehmen einer Probe erstmals, d.h. nach dem ersten Schritt einer Stechbewegung mit einem Stechelement und der Überprüfung, ob die dabei gewonnene Probenmenge ausreicht, eine für die Durchführung der Analyse zu geringe Probenmenge erkannt wird.

11. Stechgerät (2) nach Anspruch 10, **dadurch gekennzeichnet, dass** die Kontrolleinrichtung eine Signaleinheit zum Signalisieren einer für die Durchführung der Analyse zu geringen Probenmenge an den Benutzer umfasst und das mindestens eine analytische Verbrauchsmittel automatisch in die Kontaktposition verfahren wird, wenn die Kontrolleinrichtung eine für die Durchführung der Analyse zu geringe Probenmenge erkennt.

12. Stechgerät (2) nach einem der vorhergehenden Ansprüche, **dadurch gekennzeichnet, dass** das Stechelement (6) zum Durchführen eines weiteren Einstichvorgangs mit dem Stechelement (6) in die Ausgangs- oder eine Bereitschaftsposition verfahrbar ist, wenn bei der Verwendung des Stechelements (6) zum Entnehmen einer Probe erstmals, d.h. nach dem ersten Schritt einer Stechbewegung mit einem Stechelement und der Überprüfung, ob die dabei gewonnene, aufgenommene Probenmenge ausreicht, eine für die Durchführung der Analyse oder Diagnose zu geringe Probenmenge erkannt wird, und der Antrieb (5) derart ausgebildet ist, dass nach dem Durchführen des weiteren Einstichvorgangs in dem weiteren Schritt mindestens eines der analytischen Verbrauchsmittel in eine Kontaktposition verfahrbar ist, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe, die durch manuelles Melken der Probenentnahmestelle im Bereich der Einstichwunde gewonnen wurde, mit der Probe in Kontakt bringbar ist, wenn die gemäß dem Ergebnis der mit der Kontrolleinrichtung durchgeführten Überprüfung die bei dem vorausgegangenen weiteren Einstichvorgang gewonnene Probenmenge nicht zum Durchführen der Analyse ausreicht.

13. Stechgerät (2) nach dem vorhergehenden Anspruch, **dadurch gekennzeichnet, dass** der Antrieb (5) derart ausgebildet ist, dass der weitere Einstichvorgang mit einer vergrößerten Einstichtiefe durchführbar ist.

14. Analysehandgerät (1) zum Analysieren eines medizinisch bedeutsamen Bestandteils einer Körperflüssigkeit, **dadurch gekennzeichnet, dass** es ein Stechgerät (2) nach einem der vorhergehenden Ansprüche umfasst und dass in das Analysegerät (1) eine Analyseeinrichtung zum Durchführen einer Analyse oder Diagnose mittels der von dem Stechgerät (2) gewonnenen Probe integriert ist.

15. Verfahren zum Vorbereiten eines Stechgerätes (2) für das manuelle Aufnehmen einer Probe mit einem analytischen Verbrauchsmittel in einer Kontaktposition, in der das analytische Verbrauchsmittel während einer Ausfahrdauer im Bereich einer Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe, die im Bereich einer Einstichwunde gewonnen wurde, mit der Probe in Kontakt bringbar ist, wobei das Stechgerät zum Durchführen einer Analyse eines medizinisch bedeutsamen Bestandteils einer Probe vorgesehen ist und wobei das Stechgerät zum Erzeugen einer Einstichwunde in der Haut eines Menschen oder Tieres zum Gewinnen einer Probe einer Körperflüssigkeit für Analyse- oder Diagnosezwecke mit einem ersten analytischen Verbrauchsmittel, das ein Stechelement (6) umfasst, und zum Durchführen einer Analyse eines medizinisch bedeutsamen Bestandteils der Probe mit einem zweiten analytischen Verbrauchsmittel, das ein Testelement umfasst, ausgebildet ist, wobei das Stechgerät (2)
ein Andruckteil (4) zum Anpressen des Stechgeräts (2) an einen Körperteil, in dem mit dem Stechelement (6) an einer Probenentnahmestelle eine Einstichwunde erzeugt werden soll, aus der eine Probe entnommen werden soll,
einen Antrieb (5) zum Antreiben eines in das Stechgerät (2) eingesetzten Stechelements (6) für eine Stechbewegung des Stechelements (6) entlang eines vorbestimmten Einstechweges in eine Stechposition, wobei die Stechbewegung eine schnelle Einstichbewegung umfasst, auf die eine sich unmittelbar anschließende Rückzugsbewegung folgt,
ein Gehäuse mit einer Körperteilanlageöffnung (13) für den Körperteil, wobei das Stechelement (6) in einer Ausgangsposition in dem Gehäuse angeordnet ist und an der Körperteilanlageöffnung (13) mittels des Antriebs (5) in den angelegten Körperteil in die Stechposition einstechbar ist, und
eine Kontrolleinrichtung zum Überprüfen einer gewonnenen Probe darauf hin, ob die gewonnene Probenmenge ausreicht, um mit einem Testelement die Analyse durchzuführen, umfasst,
der Antrieb (5) derart ausgebildet ist, dass in einem ersten Schritt eine Stechbewegung mit einem Stechelement (6) zum Erzeugen einer Einstichwunde an einer Probenentnahmestelle für das Gewinnen einer Probe durchführbar ist,
das Stechgerät (2) derart ausgebildet ist, dass die in dem ersten Schritt gewonnene Probe mit dem ersten analytischen Verbrauchsmittel aufnehmbar ist, indem die gewonnene Probe mit dem ersten analytischen Verbrauchsmittel in Kontakt gebracht,
das Stechgerät (2) derart ausgebildet ist, dass im Anschluss an die Stechbewegung mit der Kontrolleinrichtung überprüfbar ist, ob die gewonnene, aufgenommene Probenmenge ausreicht, um mit einem Testelement die Analyse oder Diagnose durchzuführen,
wobei das Stechgerät (2) derart ausgebildet ist, dass die gewonnene, aufgenommene Probenmenge zum Durchführen einer Analyse zu einem Testelement transportierbar ist, **dadurch gekennzeichnet, dass**
der Antrieb (5) derart ausgebildet ist, dass in dem weiteren Schritt mindestens eines der analytischen Verbrauchsmittel in einer Ausfahrbewegung in eine Kontaktposition verfahren wird, in der es während einer Ausfahrdauer im Bereich der Körperteilanlageöffnung (13) in einer Position verbleibt, in der es von einem Benutzer des Stechgerätes (2) durch eine manuelle Positionierung des Stechgerätes (2) zum manuellen Aufnehmen einer Probe, die durch manuelles Melken der Probenentnahmestelle im Bereich der Einstichwunde gewonnen wurde, mit der Probe in Kontakt bringbar ist, wenn gemäß dem Ergebnis der mit der Kontrolleinrichtung durchgeführten Überprüfung die bei der vorausgegangenen Stechbewegung gewonnene Probenmenge nicht zum Durchführen der Analyse oder Diagnose ausreicht,
wobei sich die Ausfahrbewegung von der Einstechbewegung dadurch unterscheidet, dass das analytische Verbrauchsmittel länger in der Kontaktposition als in der Stechposition verbleibt, so dass es in der Kontaktposition während der Ausfahrdauer von dem Benutzer des Stechgerätes (2) durch eine manuelle Positionierung des Stechgerätes (2) zum manuellen Aufnehmen einer Probe, die durch manuelles Melken der Probenentnahmestelle im Bereich der Einstichwunde gewonnen wurde, mit der Probe in Kontakt bringbar ist.

## Claims

1. Lancing device (2) for generating a puncture wound in the skin of a human or animal to obtain a sample of a body fluid with a first analytical consumable which comprises a lancing element (6) for carrying out an analysis of a medically significant component of the sample with a second analytical consumable which comprises a test element, wherein the lancing device (2)
comprises a pressing member (4) for pressing the lancing device (2) against a body part in which it is intended to generate a puncture wound with the lancing element (6) at a sampling site from which a sample is to be taken,
a drive (5) for driving a lancing element (6) inserted into the lancing device (2) for a lancing movement of the lancing element (6) along a predetermined lancing path into a lancing position, wherein the lancing movement comprises a rapid puncture movement which is directly followed by a return movement,
a housing with a body part contact opening (13) for the body part, wherein the lancing element (6) is disposed in the housing in an initial position and at the body part contact opening (13) it can be pierced into the applied body part in the lancing position by means of the drive (5), and
a control device for checking a collected sample with regard to whether the amount of sample obtained is sufficient for carrying out the analysis with a test element,
the drive (5) is designed such that in a first step a lancing movement can be carried out with a lancing element (6) to generate a puncture wound at a sampling site for obtaining a sample,
the lancing device (2) is designed such that the sample obtained in the first step can be taken up by the first analytical consumable in that the sample obtained is brought into contact with the first analytical consumable,
the lancing device (2) is designed such that after the lancing movement the control device can be used to check whether the amount of sample obtained, taken up is sufficient to carry out the analysis with a test element,
wherein the lancing device (2) is designed such that the sample obtained, taken up can be transported to a test element for carrying out an analysis,
**characterised in that**
the drive (5) is designed in such a manner that in a further step at least one of the analytical consumables can be moved in an extending movement into a contact position in which it remains in a position in the region of the body part contact opening (13) during a deployment period, in which it can be brought into contact with the sample by a user of the lancing device (2) by a manual positioning of the lancing device (2) to manually take up a sample which has been obtained by manually milking the sampling site in the region of the puncture wound, if according to the result of the check carried out by the control device, the amount of sample obtained in the previous lancing movement is not sufficient to carry out the analysis,
wherein the extending movement differs from the puncture movement **in that** the analytical consumable remains longer in the contact position than in the lancing position such that in the contact position it can be brought into contact with the sample by the user of the lancing device (2) during the deployment period by a manual positioning of the lancing device (2) to manually take up a sample which has been obtained by manually milking the sampling site in the region of the puncture wound.

2. Lancing device (2) according to claim 1, **characterised in that** the at least one analytical consumable protrudes from an exit opening (10) of the lancing device (2) during the deployment period in the further step.

3. Lancing device (2) according to any of the preceding claims, **characterised in that** the contact position of the analytical consumable in the further step of the extending movement is different from the lancing position that it adopts in the first step of the lancing movement.

4. Lancing device (2) according to the preceding claim, **characterised in that** the drive (5) is designed such that the analytical consumable is moved further towards the sampling site or further out of the lancing device (2) in the extending movement than in the lancing movement.

5. Lancing device (2) according to any of the preceding claims, **characterised in that** the at least one analytical consumable which is moved into the contact position in the further step is an analytical consumable which comprises a lancing element (6) or a test element or an integrated disposable which comprises a lancing element (6) for carrying out the puncture process to obtain the sample of body fluid as well as a test element to carry out the analysis of a medically significant component of the sample.

6. Lancing device according to any of the preceding claims, **characterised in that** the first and the second analytical consumable are integrated in an analytical consumable which is an integrated disposable that comprises a lancing element (6) for carrying out the puncture process to obtain the sample of a body fluid as well as a test element for carrying out the analysis of a medically significant component of the sample.

7. Lancing device (2) according to any of the preceding claims, **characterised in that** the control device is designed for a visual check of the amount of sample by the user of the device.

8. Lancing device (2) according to any of the preceding claims, **characterised in that** the control device comprises an automatic sample quantity detection device or underdosing detection device.

9. Lancing device (2) according to any of the preceding claims, **characterised in that** it comprises an automatic operating mode in which the first step of a lancing movement with a lancing element (6) can be performed to generate a puncture wound at a sampling site for obtaining a sample and after the lancing movement the control device can check whether the amount of sample obtained, taken up is sufficient to carry out the analysis with a test element, and it has a manual operating mode in which in the further step at least one of the analytical consumables can be moved by means of the drive (5) into the contact position either automatically or by a trigger command of the user in which it remains in a position in the region of the body part contact opening (13) during a deployment period in which it can be brought into contact with the sample by a user of the lancing device (2) by a manual positioning of the lancing device (2) to manually take up a sample which has been obtained by manually milking the sampling site in the region of the puncture wound, if according to the result of the check carried out with the control device, the amount of sample obtained in the previous lancing movement is not sufficient to carry out the analysis.

10. Lancing device (2) according to any of the preceding claims, **characterised in that** the at least one analytical consumable can be moved into the contact position by means of the drive (5) if for the first time an inadequate amount of sample for carrying out the analysis is detected when the lancing element (6) is used to take a sample, i.e. after the first step of a lancing movement with a lancing element and the check whether the amount of sample obtained thereby is sufficient.

11. Lancing device (2) according to claim 10, **characterised in that** the control device comprises a signal unit for signaling to the user an inadequate amount of sample for carrying out the analysis and that the at least one analytical consumable is automatically moved into the contact position when the control device detects an insufficient amount of sample for carrying out the analysis.

12. Lancing device (2) according to any of the preceding claims, **characterised in that** the lancing element (6) can be moved into the initial or standby position to carry out a further puncture operation with the lancing element (6) when for the first time in the use of the lancing element (6) to remove a sample, i.e. after the first step of a lancing movement with a lancing element and the check whether the amount of sample obtained, taken up thereby is sufficient, an amount of sample that is too small to carry out the analysis or diagnosis is detected, and the drive (5) is designed such that after carrying out the further puncture process in the further step at least one of the analytical consumables can be moved into a contact position in which it remains in a position in the region of the body part contact opening (13) during a deployment period in which it can be brought into contact with the sample by a user of the lancing device (2) by a manual positioning of the lancing device (2) for manually taking up a sample which has been obtained by manually milking the sampling site in the region of the puncture wound, if according to the result of the check carried out with the control device, the amount of sample obtained in the previous further lancing process is not sufficient to carry out the analysis.

13. Lancing device (2) according to any of the preceding claims, **characterised in that** the drive (5) is designed such that the subsequent puncture process can be carried out with an increased puncture depth.

14. Hand-held analysis device (1) for analyzing a medically significant component of a body fluid, **characterised in that** it comprises a lancing device (2) according to any of the preceding claims and that an analysis apparatus for carrying out an analysis or diagnosis using the sample obtained by the lancing device (2) is integrated into the analysis device (1).

15. Method for preparing a lancing device (2) for manually taking up a sample by an analytical consumable in a contact position in which the analytical consumable remains in a position in the region of a body part contact opening (13) during a deployment period in which it can be brought into contact with the sample by a user of the lancing device (2) by a manual positioning of the lancing device (2) for a manual uptake of a sample which was obtained in the region of a puncture wound, wherein the lancing device is provided for carrying out an analysis of a medically significant component of a sample and wherein the lancing device is designed to generate a puncture wound in the skin of a human or animal for obtaining a sample of a body fluid for analytical or diagnostic purposes using a first analytical consumable which comprises a lancing element (6) and for carrying out an analysis of a medically significant component of the sample using a second analytical consumable which comprises a test element, wherein the lancing device (2)
comprises a pressing member (4) for pressing the lancing device (2) against a body part in which it is intended to generate a puncture wound with the lancing element (6) at a sampling site from which a sample is to be taken,
a drive (5) for driving a lancing element (6) inserted into the lancing device (2) for a lancing movement of the lancing element (6) along a predetermined lancing path into a lancing position, wherein the lancing movement comprises a rapid puncture movement which is directly followed by a return movement,
a housing with a body part contact opening (13) for the body part, wherein the lancing element (6) is disposed in the housing in an initial position and at the body part contact opening (13) it can be pierced into the applied body part in the lancing position by means of the drive (5), and
a control device for checking a collected sample with regard to whether the collected amount of sample is sufficient for carrying out the analysis with a test element,
the drive (5) is designed such that in a first step a lancing movement can be carried out with a lancing element (6) to generate a puncture wound at a sampling site for obtaining a sample,
the lancing device (2) is designed such that the sample obtained in the first step can be taken up by the first analytical consumable in that the sample obtained is brought into contact with the first analytical consumable,
the lancing device (2) is designed such that after the lancing movement the control device can be used to check whether the amount of sample obtained, taken up is sufficient to carry out the analysis or diagnosis with a test element,
wherein the lancing device (2) is designed such that the sample obtained, taken up can be transported to a test element for carrying out an analysis,
**characterised in that**
the drive (5) is designed in such a manner that in the further step at least one of the analytical consumables can be moved in an extending movement into a contact position in which it remains in a position in the region of the body part contact opening (13) during a deployment period, in which it can be brought into contact with the sample by a user of the lancing device (2) by a manual positioning of the lancing device (2) to manually take up a sample which has been obtained by manually milking the sampling site in the region of the puncture wound, if according to the result of the check carried out by the control device, the amount of sample obtained in the previous lancing movement is not sufficient for carrying out the analysis or diagnosis,
wherein the extending movement differs from the puncture movement **in that** the analytical consumable remains longer in the contact position than in the lancing position such that in the contact position it can be brought into contact with the sample by the user of the lancing device (2) during the deployment period by a manual positioning of the lancing device (2) to manually take up a sample which has been obtained by manually milking the sampling site in the region of the puncture wound.

## Revendications

1. Appareil de piquage (2) destiné à créer une piqûre dans la peau d'un humain ou d'un animal afin d'obtenir un échantillon d'un liquide corporel avec un premier consommable analytique, qui comprend un élément de piquage (6), destiné à mettre en oeuvre une analyse d'un composant médicalement significatif de l'échantillon avec un deuxième consommable analytique, qui comprend un élément de test, dans lequel l'appareil de piquage (2) comprend
une pièce de pression (4) destiné à presser l'appareil de piquage (2) sur une partie corporelle, dans laquelle une piqûre, à partir de laquelle un échantillon doit être prélevé, doit être créée au niveau d'un point de prélèvement d'échantillon grâce à l'élément de piquage (6),
un dispositif d'entraînement (5) destiné à entraîner un élément de piquage (6) inséré dans l'appareil de piquage (2) pour un mouvement de piquage de l'élément de piquage (6) le long d'un trajet de piquage prédéfini dans une position de piquage, dans lequel le mouvement de piquage comprend un mouvement de piquage rapide qui est suivi par un mouvement de rappel immédiatement consécutif,
une enveloppe avec une ouverture de mise en place de partie corporelle (13) destinée à la partie corporelle, dans lequel l'élément de piquage (6) est agencé dans l'enveloppe dans une position de sortie et peut être enfoncé dans la position de piquage, dans la partie corporelle mise en place, au niveau de l'ouverture de mise en place de partie corporelle (13) et au moyen du dispositif d'entraînement (5), et
un appareil de contrôle destiné à, pour un échantillon obtenu, vérifier si la quantité d'échantillon obtenue suffit pour mettre en oeuvre l'analyse avec un élément de test,
le dispositif d'entraînement (5) est réalisé de sorte que, dans une première étape, un mouvement de piquage avec un élément de piquage (6) destiné à créer une piqûre au niveau d'un point de prélèvement d'échantillon peut être mis en oeuvre pour l'obtention d'un échantillon,
l'appareil de piquage (2) est réalisé de sorte que l'échantillon obtenu lors de la première étape peut être prélevé avec le premier consommable analytique, en mettant en contact l'échantillon obtenu avec le premier consommable analytique,
l'appareil de piquage (2) est réalisé de sorte que, consécutivement au mouvement de piquage, il est possible de vérifier, avec l'appareil de contrôle, si la quantité d'échantillon prélevée obtenue est suffisante pour mettre en oeuvre l'analyse avec un élément de test,
dans lequel l'appareil de piquage (2) est réalisé de sorte que la quantité d'échantillon prélevée obtenue peut être transportée vers un élément de test en vue de la mise en oeuvre d'une analyse, **caractérisé en ce que**
le dispositif d'entraînement (5) est réalisé de sorte que, lors d'une étape ultérieure, au moins un des consommables analytiques peut être déplacé, avec un mouvement de sortie, jusqu'à une position de contact dans laquelle il reste, pendant une durée de sortie, dans le secteur de l'ouverture de mise en place de partie corporelle (13) dans une position dans laquelle il peut être mis en contact avec l'échantillon par un utilisateur de l'appareil de piquage (2) grâce un positionnement manuel de l'appareil de piquage (2) en vue du prélèvement manuel d'un échantillon obtenu par trayage manuel du point de prélèvement d'échantillon dans le secteur de la piqûre, lorsque la quantité d'échantillon obtenue lors du mouvement de piquage précédent n'est pas suffisante pour une mise en oeuvre de l'analyse, conformément au résultat de la vérification mise en oeuvre par l'appareil de contrôle,
dans lequel le mouvement de sortie se différencie du mouvement de piquage par le fait que le consommable analytique reste plus longtemps dans la position de contact que dans la position de piquage, de sorte qu'il peut être mis en contact avec l'échantillon dans la position de contact pendant la durée de sortie par l'utilisateur de l'appareil de piquage (2) grâce à un positionnement manuel de l'appareil de piquage (2) en vue du prélèvement manuel d'un échantillon obtenu par trayage manuel du point de prélèvement d'échantillon dans le secteur de la piqûre.

2. Appareil de piquage (2) selon la revendication 1, **caractérisé en ce que**, dans l'étape ultérieure, le au moins un consommable analytique fait saillie hors d'une ouverture de sortie (10) de l'appareil de piquage (2) pendant la durée de sortie.

3. Appareil de piquage (2) selon l'une quelconque des revendications précédentes, **caractérisé en ce que** la position de contact du consommable analytique dans l'étape ultérieure du mouvement de sortie est différente de la position de piquage qu'il adopte dans la première étape du mouvement de piquage.

4. Appareil de piquage (2) selon la revendication précédente, **caractérisé en ce que** le dispositif d'entraînement (5) est réalisé de sorte que le consommable analytique, lors du mouvement de sortie, est déplacé plus avant sur le point de prélèvement d'échantillon ou bien est sorti de l'appareil de piquage (2) plus loin que lors du mouvement de piquage.

5. Appareil de piquage (2) selon l'une quelconque des revendications précédentes, **caractérisé en ce que** le au moins un consommable analytique qui est déplacé dans la position de contact lors de l'étape ultérieure est un consommable analytique qui comprend un élément de piquage (6) ou un élément de test ou un dispositif jetable intégré comprenant aussi bien un élément de piquage (6) destiné à mettre en oeuvre l'action de piquage pour l'obtention de l'échantillon d'un fluide corporel qu'un élément de test destiné à mettre en oeuvre l'analyse d'un composant médicalement significatif de l'échantillon.

6. Appareil de piquage selon l'une quelconque des revendications précédentes, **caractérisé en ce que** les premier et deuxième consommables analytiques sont intégrés dans un consommable analytique qui est un dispositif jetable intégré comprenant aussi bien un élément de piquage (6) destiné à mettre en oeuvre l'action de piquage pour l'obtention de l'échantillon d'un fluide corporel qu'un élément de test destiné à mettre en oeuvre l'analyse d'un composant médicalement significatif de l'échantillon.

7. Appareil de piquage (2) selon l'une quelconque des revendications précédentes, **caractérisé en ce que** l'appareil de contrôle est réalisé en vue d'un contrôle visuel de la quantité d'échantillon par l'utilisateur de l'appareil.

8. Appareil de piquage (2) selon l'une quelconque des revendications précédentes, **caractérisé en ce que** l'appareil de contrôle comprend un appareil automatique de détermination de quantité d'échantillon ou un appareil automatique de détermination de sous-dosage.

9. Appareil de piquage (2) selon l'une quelconque des revendications précédentes, **caractérisé en ce qu'**il comprend un mode de fonctionnement automatique dans lequel la première étape d'un mouvement de piquage avec un élément de piquage (6) destiné à créer une piqûre au niveau d'un point de prélèvement d'échantillon pour l'obtention d'un échantillon peut être mise en oeuvre et dans lequel, suite au mouvement de piquage, il est possible de mettre en oeuvre, avec l'appareil de contrôle, la vérification du fait que la quantité d'échantillon prélevée obtenue est suffisante pour mettre en oeuvre l'analyse avec un élément de test, et qu'il présente un mode de fonctionnement manuel dans lequel, dans l'étape ultérieure, au moins un des consommables analytiques peut être déplacé au moyen du dispositif d'entraînement (5), de manière automatique ou grâce à un ordre de déclenchement de l'utilisateur, dans la position de contact dans laquelle il reste pendant une durée de sortie dans le secteur de l'ouverture de mise en place de partie corporelle (13) dans une position dans laquelle il peut être mis en contact, par un utilisateur de l'appareil de piquage (2) grâce à un positionnement manuel de l'appareil de piquage (2), avec l'échantillon en vue d'un prélèvement manuel d'un échantillon obtenu par trayage manuel du point de prélèvement d'échantillon dans le secteur de la piqûre, lorsque la quantité d'échantillon obtenue lors du mouvement de piqûre précédent n'est pas suffisante pour mettre en oeuvre l'analyse, conformément au résultat de la vérification mise en oeuvre par l'appareil de contrôle.

10. Appareil de piqûre (2) selon l'une quelconque des revendications précédentes, **caractérisé en ce que** le au moins un consommable analytique du dispositif d'entraînement (5) peut être déplacé dans la position de contact, lorsqu'il est déterminé qu'une quantité d'échantillon est trop faible pour la mise en oeuvre de l'analyse lors de l'utilisation de l'élément de piquage (6) pour prélever un échantillon la première fois, c'est-à-dire après la première étape d'un mouvement de piquage avec un élément de piquage et la vérification que la quantité d'échantillon obtenue est suffisante.

11. Appareil de piquage (2) selon la revendication 10, **caractérisé en ce que** l'appareil de contrôle comprend une unité de signalement destiné à signaler à l'utilisateur une quantité d'échantillon trop faible pour la mise en oeuvre de l'analyse, et le au moins un consommable analytique est déplacé de manière automatique dans la position de contact lorsque l'appareil de contrôle détermine qu'une quantité d'échantillon est trop faible pour la mise en oeuvre de l'analyse.

12. Appareil de piquage (2) selon l'une quelconque des revendications précédentes, **caractérisé en ce que** l'élément de piquage (6) destiné à mettre en oeuvre une action de piquage ultérieur avec l'élément de piquage (6) peut être déplacé dans la position de sortie ou une position de disponibilité, lorsqu'il est déterminé qu'une quantité d'échantillon est trop faible pour la mise en oeuvre de l'analyse ou du diagnostic lors de l'utilisation de l'élément de piquage (6) pour un prélèvement d'un échantillon la première fois, c'est-à-dire après la première étape d'un mouvement de piquage avec un élément de piquage et la vérification que la quantité d'échantillon prélevée obtenue est suffisante, et le dispositif d'entraînement (5) est réalisé de sorte que, après la mise en oeuvre de l'action de piquage ultérieur dans l'étape ultérieure, au moins un des consommables analytiques est déplacé dans une position de contact dans laquelle il reste pendant une durée de sortie dans le secteur de l'ouverture de mise en place de partie corporelle (13) dans une position dans laquelle il peut être mis en contact avec l'échantillon par un utilisateur de l'appareil de piquage (2) grâce à un positionnement manuel de l'appareil de piquage (2) en vue d'un prélèvement manuel d'un échantillon obtenu par trayage manuel du point de prélèvement d'échantillon dans le secteur de la piqûre, lorsque la quantité d'échantillon obtenue lors de l'action de piquage ultérieur précédente n'est pas suffisante pour mettre en oeuvre l'analyse, conformément au résultat de la vérification mise en oeuvre par l'appareil de contrôle.

13. Appareil de piquage (2) selon la revendication précédente, **caractérisé en ce que** le dispositif d'entraînement (5) est réalisé de sorte que l'action de piquage ultérieur peut être mise en oeuvre avec une profondeur de piquage augmentée.

14. Appareil manuel d'analyse (1) destiné à analyser un composant médicalement significatif d'un fluide corporel, **caractérisé en ce qu'**il comprend un appareil de piquage (2) selon l'une quelconque des revendications précédentes, et un appareil d'analyse destiné à mettre en oeuvre une analyse ou un diagnostic au moyen de l'échantillon obtenu par l'appareil de piquage (2) est intégré dans l'appareil d'analyse (1).

15. Procédé de préparation d'un appareil de piquage (2) pour le prélèvement manuel d'un échantillon avec un consommable analytique dans une position de contact dans laquelle le consommable analytique reste, pendant une durée de sortie dans le secteur d'une ouverture de mise en place de partie corporelle (13), dans une position dans laquelle il peut être mis en contact avec l'échantillon par un utilisateur de l'appareil, de piquage (2) grâce un positionnement manuel de l'appareil de piquage (2) en vue d'un prélèvement manuel d'un échantillon obtenu dans le secteur d'une piqûre, dans lequel l'appareil de piquage est prévu pour mettre en oeuvre une analyse d'un composant médicalement significatif d'un échantillon et dans lequel l'appareil de piquage est réalisé en vue de la création d'une piqûre dans la peau d'un humain ou d'un animal afin d'obtenir un échantillon d'un fluide corporel à des fins d'analyse ou de diagnostic avec un premier consommable analytique comprenant un élément de piquage (6), en vue de la mise en oeuvre d'une analyse d'un composant médicalement significatif de l'échantillon avec un deuxième consommable analytique comprenant un élément de test,
dans lequel l'appareil de piquage comprend
une pièce de pression (4) destinée à presser l'appareil de piquage (2) sur une partie corporelle dans laquelle doit être créée, au niveau d'un point de prélèvement d'échantillon et grâce à l'élément de piquage (6), une piqûre à partir de laquelle doit être prélevé un échantillon,
un dispositif d'entraînement (5) destiné à entraîner un élément de piquage (6) inséré dans l'appareil de piquage (2) pour un mouvement de piquage de l'élément de piquage (6) le long d'un trajet de piquage prédéfini dans une position de piquage, dans lequel le mouvement de piquage comprend un mouvement de piquage rapide qui est suivi par un mouvement de rappel immédiatement consécutif,
une enveloppe avec une ouverture de mise en place de partie corporelle (13) destinée à la partie corporelle, dans lequel l'élément de piquage (6) est agencé dans l'enveloppe dans une position de sortie et peut être enfoncé dans la position de piquage, dans la partie corporelle mise en place, au niveau de l'ouverture de mise en place de partie corporelle (13) et au moyen du dispositif d'entraînement (5), et
un appareil de contrôle destiné à, pour un échantillon obtenu, vérifier si la quantité d'échantillon obtenue est suffisante pour mettre en oeuvre l'analyse avec un élément de test,
le dispositif d'entraînement (5) est réalisé de sorte que, dans une première étape, un mouvement de piquage avec un élément de piquage (6) destiné à créer une piqûre au niveau d'un point de prélèvement d'échantillon peut être mis en oeuvre pour l'obtention d'un échantillon,
l'appareil de piquage (2) est réalisé de sorte que l'échantillon obtenu lors de la première étape peut être prélevé avec le premier consommable analytique en mettant en contact l'échantillon obtenu avec le premier consommable analytique,
l'appareil de piquage (2) est réalisé de sorte que, consécutivement au mouvement de piquage, il est possible de vérifier, avec l'appareil de contrôle, si la quantité d'échantillon prélevée obtenue est suffisante pour mettre en oeuvre l'analyse ou le diagnostic avec un élément de test,
dans lequel l'appareil de piquage (2) est réalisé de sorte que la quantité d'échantillon prélevée obtenue peut être transportée vers un élément de test en vue de la mise en oeuvre d'une analyse, **caractérisé en ce que**
le dispositif d'entraînement (5) est réalisé de sorte que, lors de l'étape ultérieure, au moins un des consommables analytiques est déplacé, avec un mouvement de sortie, jusqu'à une position de contact dans laquelle il reste, pendant une durée de sortie, dans le secteur de l'ouverture de mise en place de partie corporelle (13) dans une position dans laquelle il peut être mis en contact avec l'échantillon par un utilisateur de l'appareil de piquage (2) grâce à un positionnement manuel de l'appareil de piquage (2) en vue d'un prélèvement manuel d'un échantillon obtenu par trayage manuel du point de prélèvement d'échantillon dans le secteur de la piqûre, lorsque la quantité d'échantillon obtenue lors de mouvement de piquage précédent n'est pas suffisante pour une mise en oeuvre de l'analyse ou du diagnostic, conformément au résultat de la vérification mise en oeuvre par l'appareil de contrôle,
dans lequel le mouvement de sortie se différencie du mouvement de piquage par le fait que le consommable analytique reste plus longtemps dans la position de contact que dans la position de piquage, de sorte qu'il peut être mis en contact avec l'échantillon dans la position de contact pendant la durée de sortie par l'utilisateur de l'appareil de piquage (2) grâce un positionnement manuel de l'appareil de piquage (2) en vue du prélèvement manuel d'un échantillon obtenu par trayage manuel du point de prélèvement d'échantillon dans le secteur de la piqûre.
